# FASCICULE DE BREVET EUROPEEN

(11) **EP 0 624 195 B1**
(45) Date de publication et mention de la délivrance du brevet: **15.09.2004**
(21) Numéro de dépôt: 93904129.9
(22) Date de dépôt: 28.01.1993
(51) Int. Cl.: C12N 15/12, C12N 15/62, C12N 15/81, C12P 21/02, C12N 1/19

(54) **NOUVEAUX POLYPEPTIDES BIOLOGIQUEMENT ACTIFS, LEUR PREPARATION ET COMPOSITION PHARMACEUTIQUE LES CONTENANT**
NEUARTIGE, BIOLOGISCH AKTIVE POLYPEPTIDE, IHRE HERSTELLUNG UND DIESE POLYPEPTIDE ENTHALTENDE PHARMAZEUTISCHE ZUSAMMENSETZUNG
NOVEL BIOLOGICALLY ACTIVE POLYPEPTIDES, PREPARATION THEREOF AND PHARMACEUTICAL COMPOSITION CONTAINING SAID POLYPEPTIDES

(30) Priorité: 31.01.1992 FR 9201064
(43) Date de publication de la demande: 17.11.1994
(60) Demande divisionnaire: 04075986.2
(73) Titulaire: Delta Biotechnology Limited, Nottingham NG7 1FD (GB)
(72) Inventeur: FLEER, Reinhard, F-91440 Bures-sur-Yvette (FR); FOURNIER, Alain, F-92000 Châtenay-Malabry (FR); GUITTON, Jean-Dominique, F-75013 Paris (FR); JUNG, Gérard, F-91310 Montlhéry (FR); YEH, Patrice, F-75005 Paris (FR)
(74) Mandataire: Bassett, Richard Simon
(86) Numéro de dépôt international: PCT/FR1993/000085
(87) Numéro de publication internationale: WO 1993/015199

(56) Documents cités:
- EP-A- 0 413 622
- WO-A-89/02922
- WO-A-90/13653
- WO-A-93/00437
- DATABASE WPIL Section Ch, Week 9141, Derwent Publications Ltd., London, GB; Class C, AN 91-300976

## Description

La présente invention concerne de nouveaux polypeptides biologiquement actifs, leur préparation et des compositions pharmaceutiques les contenant.

Plus particulièrement, la présente invention concerne des polypeptides recombinants essentiellement composés d'une partie active dérivée d'un polypeptide, naturel ou artificiel, ayant une activité thérapeutique, et couplé à une albumine ou à un variant de l'albumine. Il est entendu que l'activité thérapeutique des polypeptides de l'invention peut être soit directe (traitement des maladies), ou indirecte (et par exemple utilisable dans la prévention des maladies, dans la conception des vaccins, dans les techniques de l'imagerie médicale etc...).

Il est entendu dans ce qui suit que les variants de l'albumine désignent toute protéine à haute demie-vie plasmatique obtenue par modification (mutation, délétion et/ou addition) par les techniques du génie génétique d'un gène codant pour un isomorphe donné de la sérum-albumine humaine, ainsi que toute macromolécule à haute demie-vie plasmatique obtenue par modification in vitro de la protéine codée par de tels gènes. L'albumine étant très polymorphe, de nombreux variants naturels ont été identifiés et répertoriés [Weitkamp L.R. et al., Ann. Hum. Genet. 37 (1973) 219].

Le but de la présente invention est d'élaborer des protéines artificielles biologiquement actives et utilisables sur le plan pharmaceutique. En effet, de nombreux polypeptides possédant une ou plusieurs activités thérapeutiques potentielles ne peuvent être exploités pharmaceutiquement. Ceci peut avoir différentes raisons, telles que notamment leur faible stabilité in vivo, leur structure complexe ou fragile, la difficulté de les produire à une échelle industriellement acceptable, etc... De même, certains polypeptides ne donnent pas les résultats attendus in vivo en raison de problèmes d'administration, de conditionnement, de pharmacocinétique etc...

EP 413 622 divulgue la fusion de CD4 soluble à l'albumine sérique. WO 90/13653 divulgue certains composants particuliers de fusion à l'albumine, pour les objectifs d'une sécrétion améliorée tout particulièrement. WO 89/02922 divulgue la fusion de CD4 soluble à une molécule apparentée du point de vue de sa structure, à savoir sa fusion à une chaîne d'immunoglobulines (Ig).

La présente invention permet de remédier à ces inconvénients. La présente invention fournit en effet de nouvelles molécules permettant une exploitation optimale sur le plan thérapeutique des propriétés biologiques de ces polypeptides. La présente invention résulte notamment de la mise en évidence qu'il est possible de coupler génétiquement toute structure active dérivée d'un polypeptide biologiquement actif à une autre structure protéique constituée d'albumine, sans en altérer lesdites propriétés biologiques. Elle résulte également de la mise en évidence par la demanderesse que la sérum-albumine humaine permet de présenter efficacement la structure active à ses sites d'interaction, et qu'elle assure une stabilité plasmatique élevée du polypeptide de l'invention. Les polypeptides de l'invention permettent ainsi de maintenir dans l'organisme une activité biologique donnée pendant un temps prolongé. Ils permettent ainsi de réduire les doses administrées et, dans certains cas, de potentialiser l'effet thérapeutique, par exemple en réduisant les effets secondaires consécutifs à une administration plus importante. Les polypeptides de l'invention permettent de plus de générer et d'utiliser des structures dérivées des polypeptides biologiquement actifs très petites et donc très spécifiques d'un effet recherché. Il est entendu que les peptides ayant une activité biologique présentant un intérêt thérapeutique peuvent également correspondre à des séquences peptidiques non naturelles, isolées par exemple à partir de banques peptidiques aléatoires. Les polypeptides de l'invention possèdent par ailleurs une répartition particulièrement avantageuse dans l'organisme, ce qui modifie leurs propriétés pharmacocinétiques et favorise le développement de leur activité biologique et leur utilisation. En outre, ils présentent également l'avantage d'être faiblement ou non-immunogéniques pour l'organisme dans lequel ils sont utilisés. Finalement, les polypeptides de l'invention peuvent être exprimés (et préférentiellement sécrétés) par des organismes recombinants, à des niveaux permettant leurs exploitation industrielle.

Un objet de la présente invention concerne donc des polypeptides comportant une partie active dérivée d'un polypeptide ayant une activité thérapeutique, couplée à une albumine ou à un variant de l'albumine.

Dans un mode de réalisation particulier, les peptides possédant une activité thérapeutique ne sont pas d'origine humaine.

Plus particulièrement, dans les molécules de l'invention, le polypeptide ayant une activité thérapeutique est un polypeptide d'origine humaine ou un variant moléculaire. Il s'agit de tout ou partie d'une hormone, d'un interféron, d'une interleukine, érythropoiétine, G-CSF, ou l'insuline.

La partie active des polypeptides de l'invention peut être constituée, par exemple, par le polypeptide ayant une activité thérapeutique entier, ou par une structure dérivée de celui-ci, ou encore par un polypeptide non naturel isolé à partir d'une banque peptidique. Au sens de la présente invention, on entend par structure dérivée tout polypeptide obtenu par modification et conservant une activité thérapeutique. Par modification, on doit entendre toute mutation, substitution, délétion, addition ou modification de nature génétique et/ou chimique. De tels dérivés peuvent être générés dans des buts différents, tels que notamment celui d'augmenter l'affinité de la molécule pour ses sites de fixation, celui d'améliorer ses niveaux de production, celui d'augmenter sa résistances aux protéases, celui d'augmenter son efficacité thérapeutique ou encore de réduire ses effets secondaires, ou celui de lui conférer de nouvelles propriétés biologiques. A titre d'exemple, les polypeptides chimères de l'invention possèdent des propriétés pharmacocinétiques et une activité biologique utilisable pour la prévention ou le traitement des maladies.

Des polypeptides de l'invention particulièrement avantageux sont ceux dans lesquels la partie active présente :
(a) la structure peptidique entière ou,
(b) une structure dérivée de (a) par modification structurale (mutation, substitution addition et/ou délétion d'un ou plusieurs résidus) et possédant une activité thérapeutique.

Parmi les structures du type (b), on peut citer plus particulièrement les molécules dans lesquelles certains sites de N- ou O-glycosylation ont été modifiés ou supprimés, les molécules dans lesquelles un ou plusieurs résidus ont été substitués, ou les molécules dans lesquelles tous les résidus cystéine ont été substitués. On peut citer également des molécules obtenues à partir de (a) par délétion de régions n'intervenant pas ou peu dans l'interaction avec les sites de .liaison considérés, ou exprimant une activité indésirable, et des molécules comportant par rapport à (a) des résidus supplémentaires, tels que par exemple une méthionine N-terminale et/ou un signal de sécrétion et/ou un peptide de jonction.

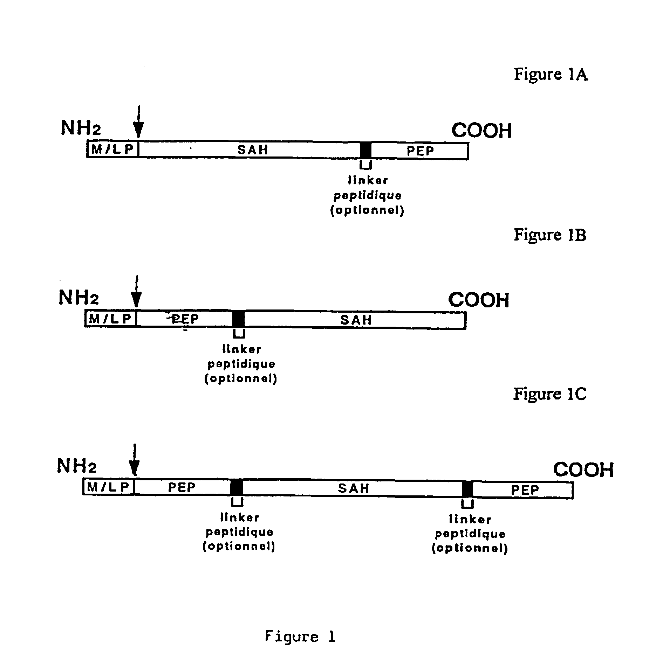

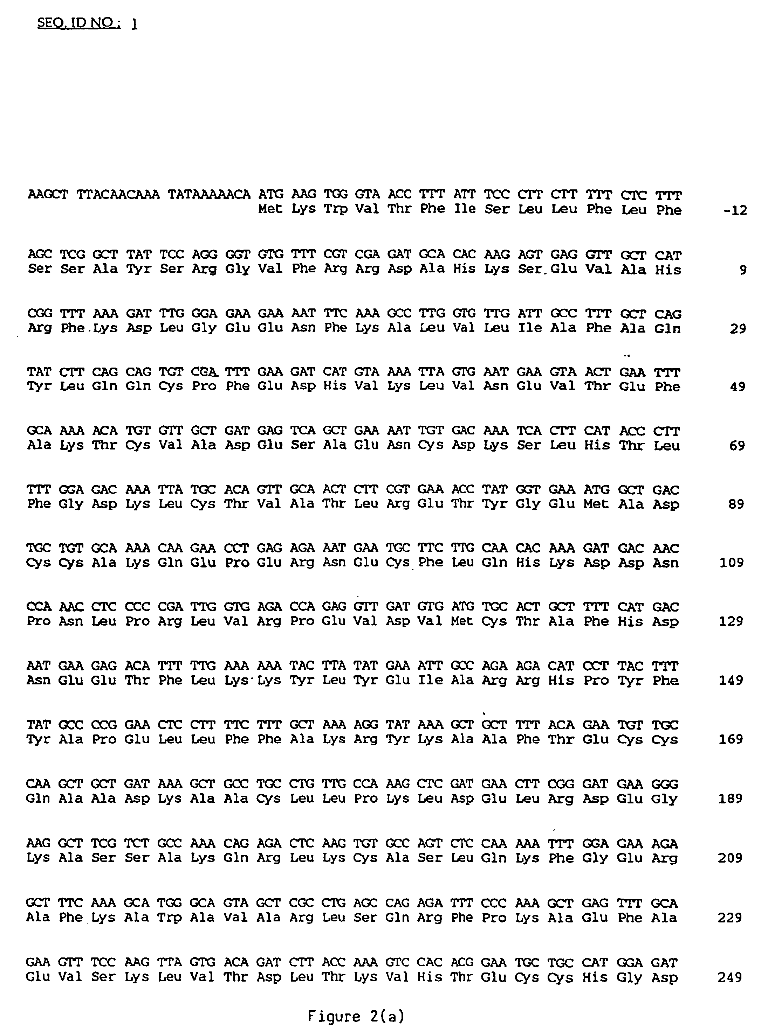

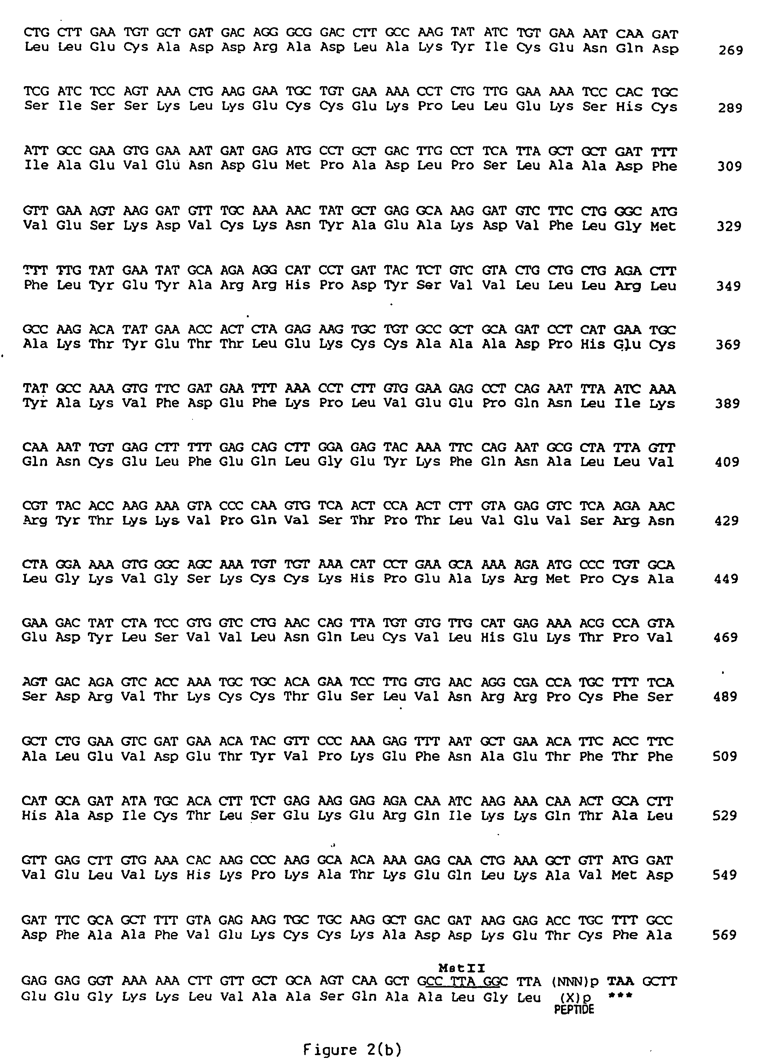

La partie active des molécules de l'invention peut être couplée soit directement soit par l'intermédiaire d'un peptide artificiel à l'albumine. De plus, elle peut constituer l'extrémité N-terminale comme l'extrémité C-terminale de la molécule. Préférentiellement, dans les molécules de l'invention, la partie active constitue la partie C-terminale de la chimère. Il est également entendu que la partie biologiquement active peut être redondante au sein de la chimère. Une représentation schématique des molécules de l'invention est donnée à la Figure 1.

Un autre objet de l'invention concerne un procédé de préparation des molécules chimères décrites ci-avant. Plus précisément, ce procédé consiste à faire exprimer par un hôte cellulaire eucaryote ou procaryote une séquence nucléotidique codant pour le polypeptide désiré, puis à récolter le polypeptide produit.

Parmi les hôtes eucaryotes utilisables dans le cadre de la présente invention, on peut citer les cellules animales, les levures, ou les champignons. En particulier, s'agissant de levures, on peut citer les levures du genre Saccharomyces, Kluyveromyces, Pichia, Schwanniomyces, ou Hansenula. S'agissant de cellules animales, on peut citer les cellules COS, CHO, C127, etc... Parmi les champignons susceptibles d'être utilisés dans la présente invention, on peut citer plus particulièrement Aspergillus ssp. ou Trichoderma ssp. Comme hôtes procaryotes, on préfère utiliser les bactéries telles que Escherichia coli, ou appartenant aux genres Corynebacterium, Bacillus, ou Streptomyces.

Les séquences nucléotidiques utilisables dans le cadre de la présente invention peuvent être préparées de différentes manières. Généralement, elles sont obtenues en assemblant en phase de lecture les séquences codant pour chacune des parties fonctionnelles du polypeptide. Celles-ci peuvent être isolées par les techniques de l'homme de l'art, et par exemple directement à partir des ARN messsagers (ARNm) cellulaires, ou par reclonage à partir d'une banque d'ADN complémentaire (ADNc), ou encore il peut s'agir de séquences nucléotidiques totalement synthétiques. Il est entendu de plus que les séquences nucléotidiques peuvent également être ultérieurement modifiées, par exemple par les techniques du génie génétique, pour obtenir des dérivés ou des variants desdites séquences.

Plus préférentiellement, dans le procédé de l'invention, la séquence nucléotidique fait partie d'une cassette d'expression comprenant une région d'initiation de la transcription (région promoteur) permettant, dans les cellules hôtes, l'expression de la séquence nucléotidique placée sous son contrôle et codant pour les polypeptides de l'invention. Cette région peut provenir de régions promoteurs de gènes fortement exprimés dans la cellule hôte utilisée, l'expression étant constitutive ou régulable. S'agissant de levures, il peut s'agir du promoteur du gène de la phosphoglycérate kinase (PGK), de la glycéraldéhyde-3-phosphate déshydrogénase (GPD), de la lactase (LAC4), des énolases (ENO), des alcools deshydrogénases (ADH), etc... S'agissant de bactéries, il peut s'agir du promoteur des gènes droit ou gauche du bactériophage lambda (P_{L}, P_{R}), ou encore des promoteurs des gènes des opérons tryptophane (Pₜᵣₚ) ou lactose (P_{lac}). En outre, cette région de contrôle peut être modifiée, par exemple par mutagénèse in vitro, par introduction d'éléments additionnels de contrôle ou de séquences synthétiques, ou par des délétions ou des substitutions des éléments originels de contrôle. La cassette d'expression peut également comprendre une région de terminaison de la transcription fonctionnelle dans l'hôte envisagé, positionnée immédiatement en aval de la séquence nucléotidique codant pour un polypeptide de l'invention.

Dans un mode préféré, les polypeptides de l'invention résultent de l'expression dans un hôte eucaryote ou procaryote d'une séquence nucléotidique et de la sécrétion du produit d'expression de ladite séquence dans le milieu de culture. Il est en effet particulièrement avantageux de pouvoir obtenir par voie recombinante des molécules directement dans le milieu de culture. Dans ce cas, la séquence nucléotidique codant pour un polypeptide de l'invention est précédée d'une séquence "leader" (ou séquence signal) dirigeant le polypeptide naissant dans les voies de sécrétion de l'hôte utilisé. Cette séquence "leader" peut être la séquence signal naturelle du polypeptide biologiquement actif dans le cas où celui-ci est une protéine naturellement sécrétée, ou celle de la structure stabilisatrice, mais il peut également s'agir de toute autre séquence "leader" fonctionnelle, ou d'une séquence "leader" artificielle. Le choix de l'une ou l'autre de ces séquences est notamment guidé par l'hôte utilisé. Des exemples de séquences signal fonctionnelles incluent celles des gènes des phéromones sexuelles ou des toxines "killer" de levures.

En plus de la cassette d'expression, un ou plusieurs marqueurs permettant de sélectionner l'hôte recombiné peuvent être additionnés, tels que par exemple le gène URA3 de la levure S. cerevisiae, ou des gènes conférant la résistance à des antibiotiques comme la généticine (G418) ou à tout autre composé toxique comme certains ions métalliques.

L'ensemble constitué par la cassette d'expression et par le marqueur de sélection peut être introduit directement dans les cellules hôtes considérées, soit inséré préalablement dans un vecteur autoréplicatif fonctionnel. Dans le premier cas, des séquences homologues à des régions présentes dans le génôme des cellules hôtes sont préférentiellement additionnées à cet ensemble; lesdites séquences étant alors positionnées de chaque côté de la cassette d'expression et du gène de sélection de façon à augmenter la fréquence d'intégration de l'ensemble dans le génôme de l'hôte en ciblant l'intégration des séquences par recombinaison homologue. Dans le cas où la cassette d'expression est insérée dans un système réplicatif, un système de réplication préféré pour les levures du genre Kluyveromyces est dérivé du plasmide pKD1 initialement isolé de K.drosophilarum; un système préféré de réplication pour les levures du genre Saccharomyces est dérivé du plasmide 2µ de S. cerevisiae. De plus, ce plasmide d'expression peut contenir tout ou partie desdits systèmes de réplication, ou peut combiner des éléments dérivés du plasmide pKD1 aussi bien que du plasmide 2µ.

En outre, les plasmides d'expression peuvent être des vecteurs navettes entre un hôte bactérien tel que Escherichia coli et la cellule hôte choisie. Dans ce cas, une origine de réplication et un marqueur de sélection fonctionnant dans l'hôte bactérien sont requises. Il est également possible de positionner des sites de restriction entourant les séquences bactériennes et uniques sur le vecteur d'expression: ceci permet de supprimer ces séquences par coupure et religature in vitro du vecteur tronqué avant transformation des cellules hôtes, ce qui peut résulter en une augmentation du nombre de copies et en une stabilité accrue des plasmides d'expression dans lesdits hôtes. Par exemple, de tels sites de restriction peuvent correspondre aux séquences telles que 5'-GGCCNNNNNGGCC-3' (SfiI) ou 5'-GCGGCCGC-3' (NotI) dans la mesure où ces sites sont extrêmement rares et généralement absents d'un vecteur d'expression.

Après construction de tels vecteurs ou cassette d'expression, ceux-ci sont introduits dans les cellules hôtes retenues selon les techniques classiques décrites dans la littérature. A cet égard, toute méthode permettant d'introduire un ADN étranger dans une cellule peut être utilisée. Il peut s'agir notamment de transformation, électroporation, conjugaison, ou toute autre technique connue de l'homme de l'art. A titre d'exemple pour les hôtes de type levure, les différentes souches de Kluyveromyces utilisées ont été transformées en traitant les cellules entières en présence d'acétate de lithium et de polyéthylène glycol, selon la technique décrite par Ito et al. [J. Bacteriol. 153 (1983) 163]. La technique de transformation décrite par Durrens et al. [Curr. Genet. 18 (1990) 7] utilisant l'éthylène glycol et le diméthylsulfoxyde a également été utilisée. Il est aussi possible de transformer les levures par électroporation, selon la méthode décrite par Karube et al. [FEBS Letters 182 (1985) 90]. Un protocole alternatif est également décrit en détail dans les exemples qui suivent.

Après sélection des cellules transformées, les cellules exprimant lesdits polypeptides sont inoculées et la récupération desdits polypeptides peut être faite, soit au cours de la croissance cellulaire pour les procédés "en continu", soit en fin de croissance pour les cultures "en lots" ("batch"). Les polypeptides qui font l'objet de la présente invention sont ensuite purifiés à partir du surnageant de culture en vue de leur caractérisation moléculaire, pharmacocinétique et biologique.

Un système d'expression préféré des polypeptides de l'invention consiste en l'utilisation des levures du genre Kluyveromyces comme cellule hôte, transformées par certains vecteurs dérivés du réplicon extrachromosomique pKD1 initialement isolé chez K. marxianus var. drosophilarum. Ces levures, et en particulier K. lactis et K. fragilis sont généralement capables de répliquer lesdits vecteurs de façon stable et possèdent en outre l'avantage d'être incluses dans la liste des organismes G.R.A.S. ("Generally Recognized As Safe"). Des levures privilégiées sont préférentiellement des souches industrielles du genre Kluyveromyces capables de répliquer de façon stable lesdits plasmides dérivés du plasmide pKD1 et dans lesquels a été inséré un marqueur de sélection ainsi qu'une cassette d'expression permettant la sécrétion à des niveaux élevés des polypeptides de l'invention.

La présente invention concerne également les séquences nucléotidiques codant pour les polypeptides chimères décrits ci-avant, ainsi que les cellules recombinantes, eucaryotes ou procaryotes, comprenant de telles séquences.

La présente invention concerne aussi l'application à titre de médicament des polypeptides selon la présente invention. Plus particulièrement, l'invention a pour objet toute composition pharmaceutique comprenant un ou plusieurs polypeptides ou séquences nucléotidiques tels que décrits ci-avant. Les séquences nucléotidiques peuvent en effet être utilisées en thérapie génique.

La présente invention sera plus complètement décrite à l'aide des exemples qui suivent, qui doivent être considérés comme illustratifs et non limitatifs.

### LISTE DES FIGURES

Les représentations des plasmides indiquées dans les Figures suivantes ne sont pas traçées à l'échelle et seuls les sites de restriction importants pour la compréhension des clonages réalisés ont été indiqués.
**Figure 1 :** Schématisation des chimères du type SAH-PEPTIDE (A), du ype PEPTTDE-SAH (B) ou PEPTIDE-SAH-PEPTIDE (C). Abréviations utilisées : M/LP, résidu méthionine initiateur de la traduction, éventuellement suivie d'une séquence signal de sécrétion; SAH, albumine mature ou un de ses variants moléculaires; PEP, peptide d'origine naturelle ou artificielle possédant une propriété thérapeutique donnée. La séquence PEP peut être présente plusieurs fois dans les molécules de type A, B ou C. La flèche noire indique l'extrémité N-terminale de la protéine mature.
   **SEQ ID n° 1:** Exemples de séquences nucléotidiques d'un fragment de restriction HindIII codant pour une protéine chimère du type prépro-SAH-PEPTIDE. Les flèches noires indiquent la fin des régions "pré" et "pro" de la SAH. Le site de restriction MstII est souligné et le codon spécifiant la terminaison de la traduction est en caractères gras.

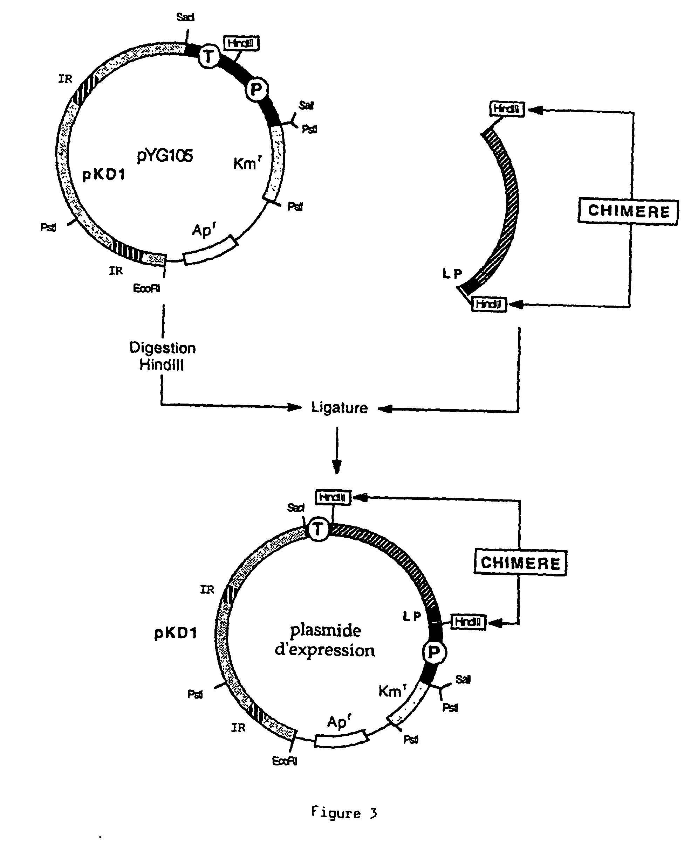

**Figure 3 :** Carte de restriction du plasmide pYG105 et stratégie générique de construction des plasmides d'expression des protéines chimères de la présente invention. Abréviations utilisées : P, promoteur transcriptionnel ; T, terminateur transcriptionnel ; IR, séquences répétées inversées du plasmide pKD1 ; LP, séquence signal de sécrétion ; Ap^{r} et Km^{r} désignent respectivement les gènes de résistance à l'ampicilline (E. coli) et au G418 (levures).

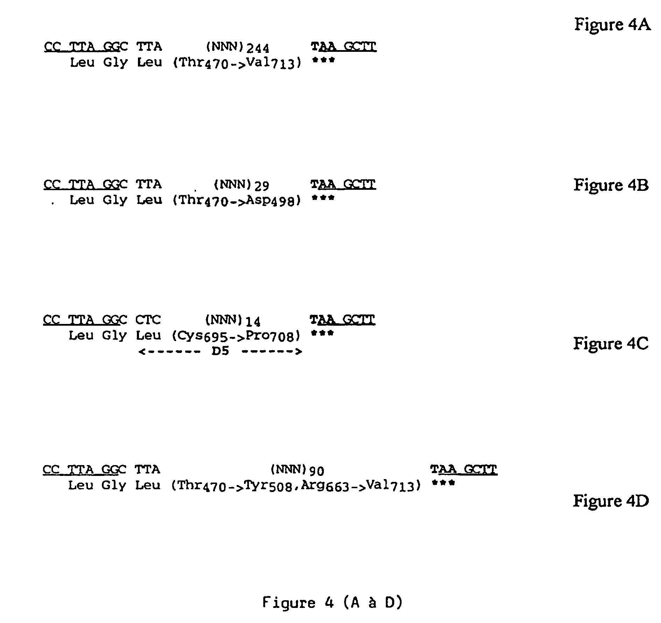

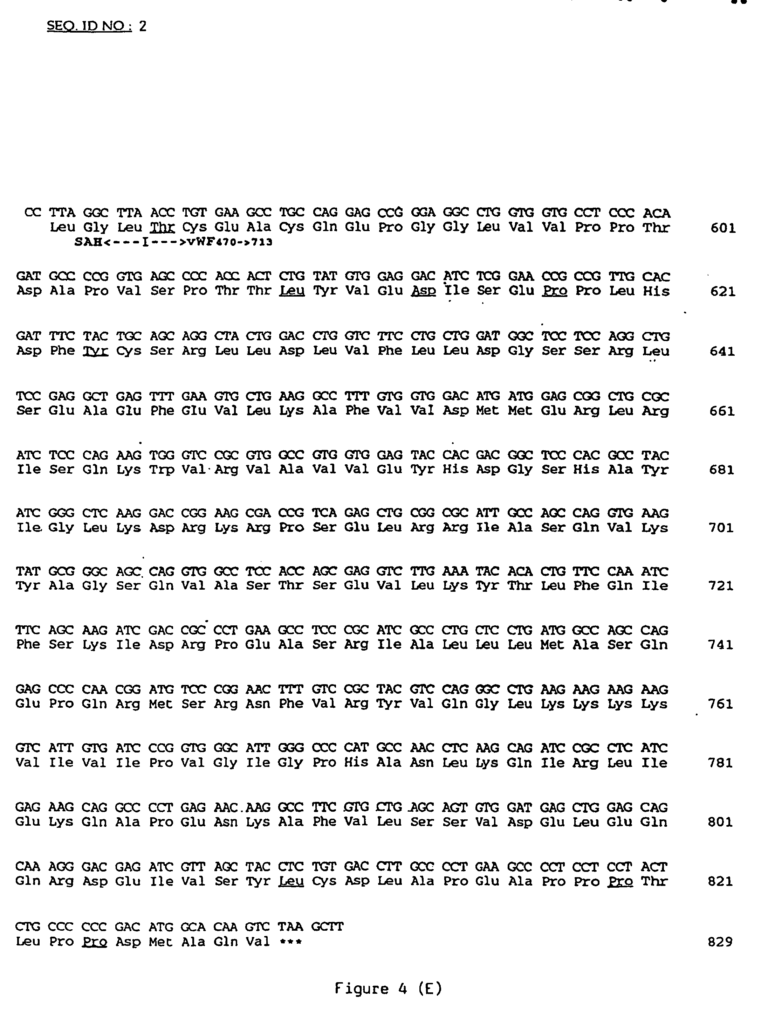

**Figure 4 :** Exemples de séquences nucléotidiques de fragments de restriction MstII-HindIII dérivés du facteur von Willebrand. Représentation de la structure des fragments MstII-HindIII des plasmides pYG1248 (panneau A), pYG1214 (panneau B), pYG1206 [panneau C, dans cette chimère particulière le résidu Leu694 du vWF est également le dernier résidu (Leu585) de la SAH] et pYG1223 (panneau D) ; la numérotation des acides aminés correspond à la numérotation du vWF mature d'après Titani et al. [Biochemistry 25 (1986) 3171-3184]. Les sites de restriction MstII et HindIII sont soulignés et le codon de terminaison de la traduction est en caractères gras. SEQ ID n° 2 : séquence nucléotidique du fragment de restriction MstII-HindIII du plasmide pYG1248. La numérotation des acides aminés (colonne de droite) correspond à la protéine chimère SAH-vWF470->713 mature (829 résidus). Les résidus Thr470, Leu494, Asp498, Pro502, Tyr508, Leu694, Pro704, et Pro708 du vWF mature sont soulignés.

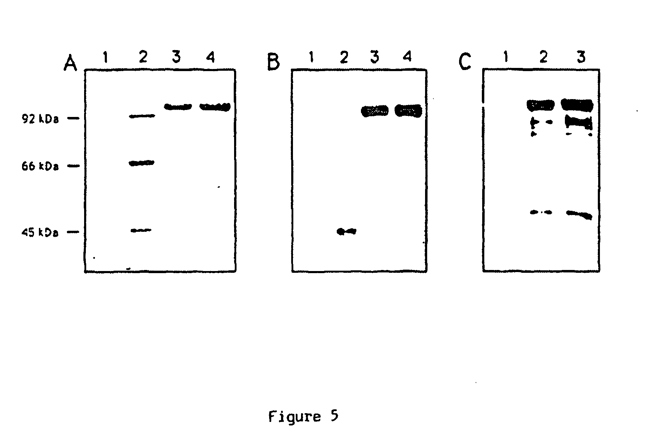

**Figure 5 :** Caractérisation du matériel sécrété après 4 jours de culture (erlenmeyers) de la souche CBS 293.91 transformée par les plasmides pYG1248 (plasmide d'expression d'une chimère du type SAH-vWF Thr470->Val713) et pKan707 (plasmide contrôle). Dans cette expérience les résultats des panneaux A, B, et C ont été migrés sur le même gel (SDS-PAGE 8,5 %) puis traités séparemment.
   **A,** coloration au bleu de coomassie; standard de poids moléculaire (piste 2); surnageant équivalent à 50 µl de la culture transformée par les plasmides pKan707 en milieu YPL (piste 1), ou pYG1248 en milieu YPD (piste 3) ou YPL (piste 4).
   **B,** caractérisation immunologique du matériel sécrété après utilisation d'anticorps de souris dirigés contre le vWF humain: même légende qu'en A à l'exception que des standards biotinilés de poids moléculaire ont été utilisés.
   **C,** caractérisation immunologique du matériel sécrété après utilisation d'anticorps de lapin dirigés contre l'albumine humaine: surnageant équivalent à 50 µl de la culture transformée par les plasmides pKan707 en milieu YPL (piste 1), ou pYG1248 en milieu YPD (piste 2) ou YPL (piste 3).

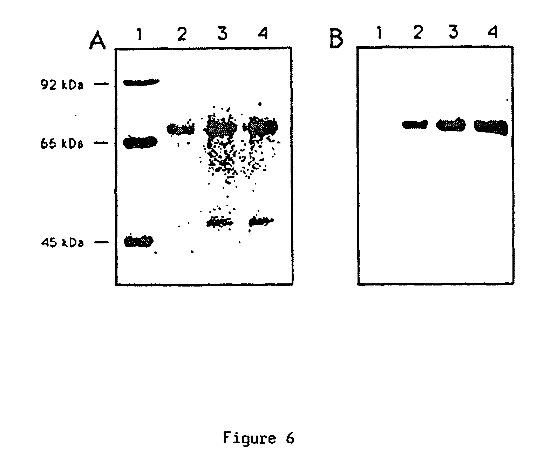

**Figure 6 :** Cinétique de sécrétion d'une chimère de l'invention par la souche CBS 293.91 transformée par le plasmide pYG1206 (SAH-vWF Leu694-Pro708).
   **A,** coloration au bleu de coomassie ; standard de poids moléculaire (piste 1) ; surnageant équivalent à 2,5 µl d'une culture "Fed Batch" en milieu YPD après 24h. (piste 2), 40h. (piste 3) ou 46h. (piste 4) de croissance.
   **B,** caractérisation immunologique du matériel sécrété après utilisation d'anticorps de souris dirigés contre le vWF humain : même légende qu'en A à l'exception que des standards biotinilés de poids moléculaire ont été utilisés.

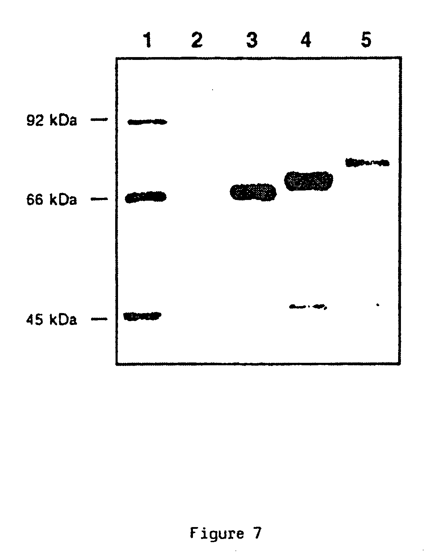

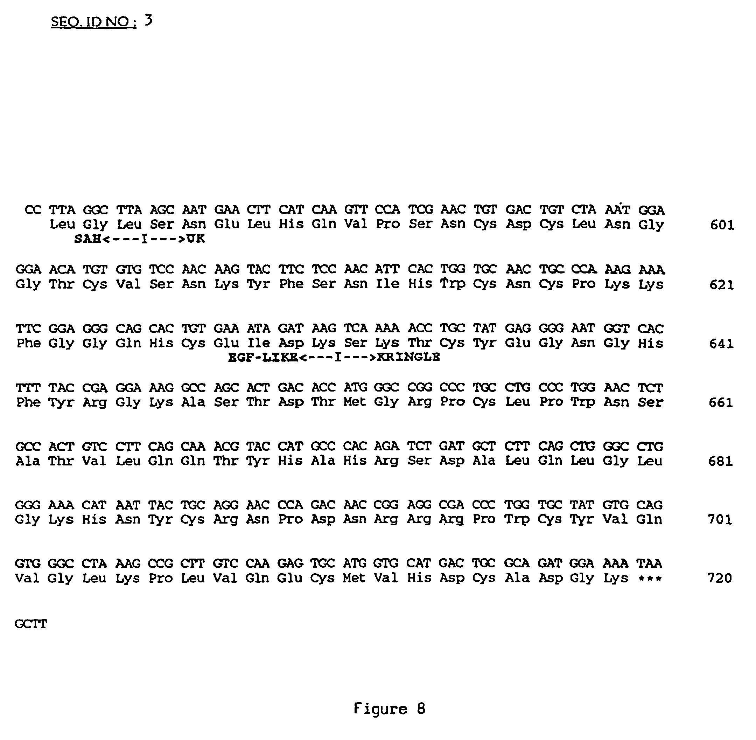

**Figure 7 :** Caractérisation du matériel sécrété par K. lactis transformé par les plasmides pKan707 (plasmide contrôle, piste 2), pYG1206 (piste 3), pYG1214 (piste 4) et pYG1223 (piste 5) ; standard de poids moléculaire (piste 1). Les dépôts correspondent à 50 µl de surnageant d'une culture stationnaire après croissance en milieu YPD, migration dans un gel à 8.5 % d'acrylamide et coloration au bleu de coomassie.
   **SEQ ID n° 3 :** Séquence nucléotidique du fragment de restriction MstII-HindIII du plasmide pYG1341 (SAH-UK1->135). La limite du domaine EGF-like (UK1->46) présent dans le fragment de restriction MstII-HindIII du plasmide pYG1340 est indiquée. La numérotation des acides aminés correspond à la protéine chimère SAH-UK1->135 mature (720 résidus).

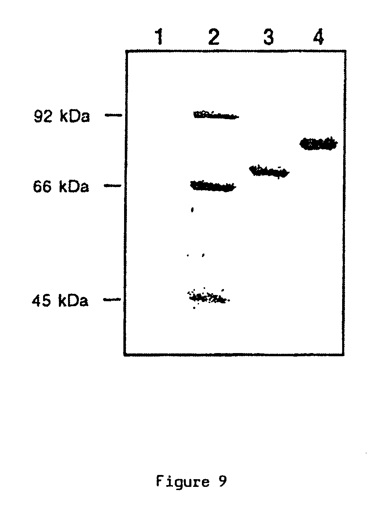

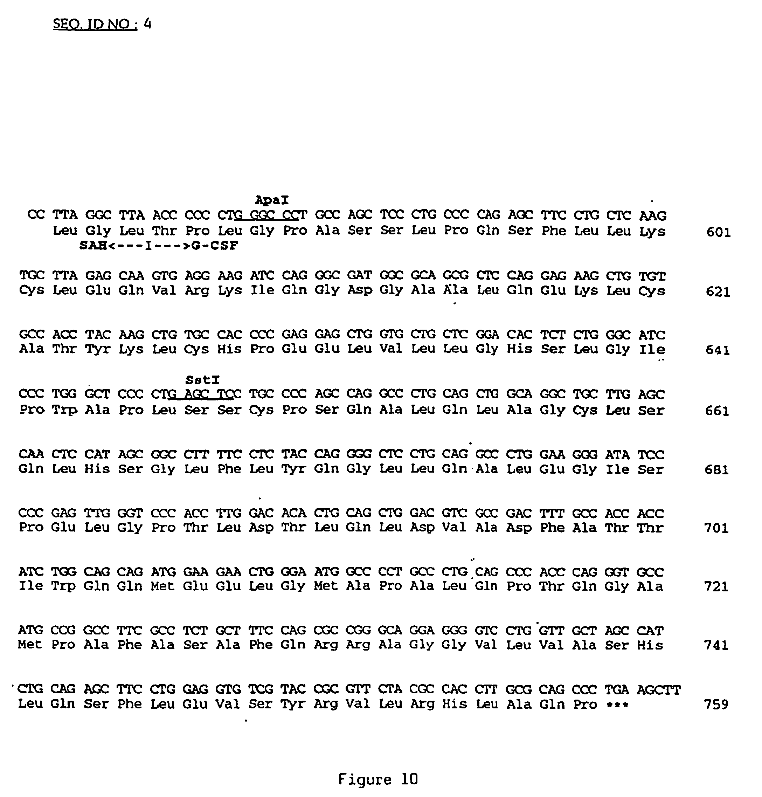

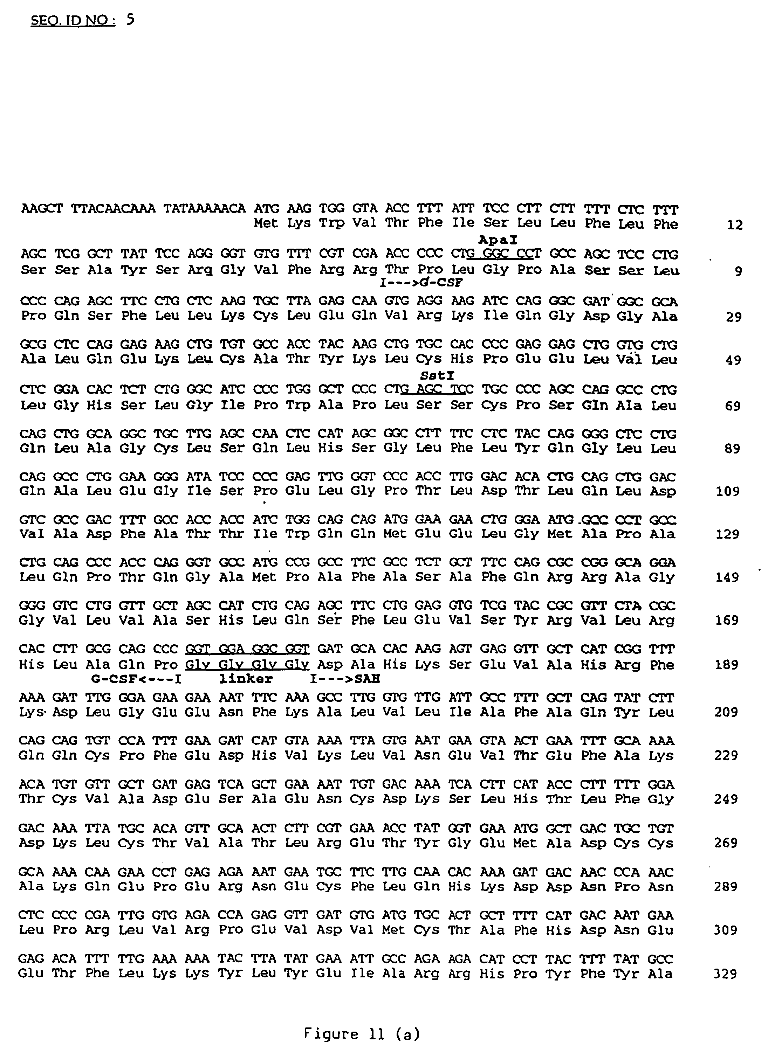

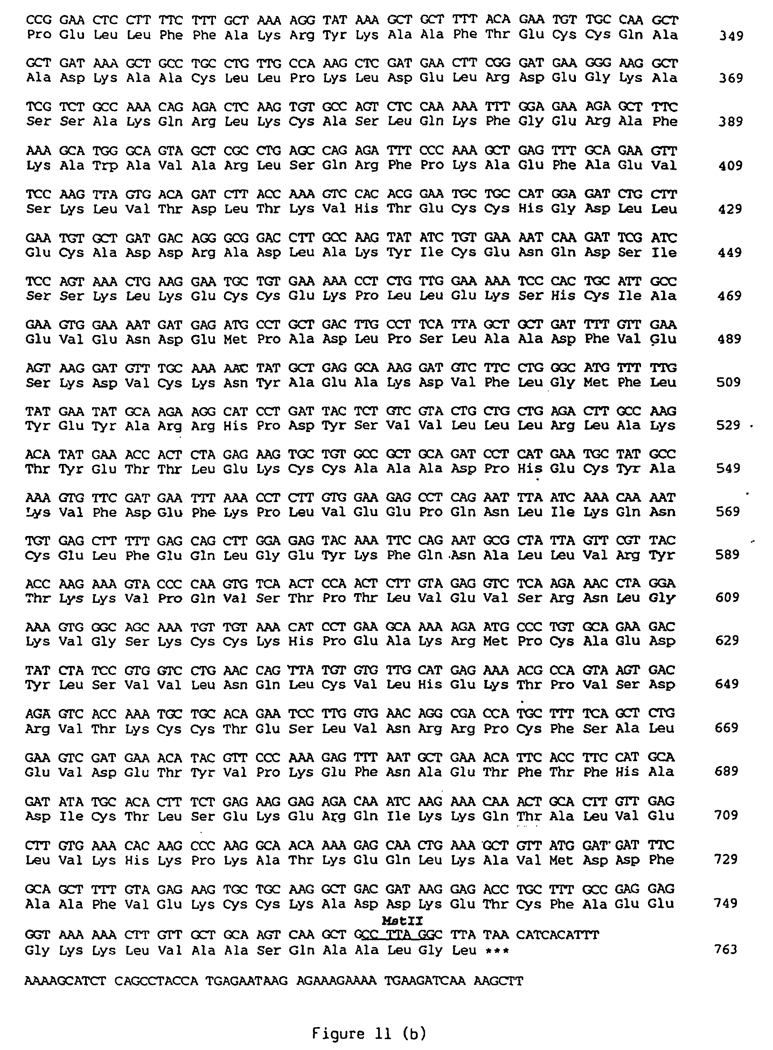

**Figure 9 :** Sécrétion des chimères SAH-UK1-46 et SAH-UK1-135 par la souche CBS 293.91 respectivement transformée par les plasmides pYG1343 (SAH-UK1-46) et pYG1345 (SAH-UK1-135), après 4 jours de croissance (milieu YPL+G418). Les dépôts (équivalents à 50 µl de culture) sont migrés en gel PAGE-SDS à 8,5 % et colorés au bleu de coomassie: surnageant d'un clone transformé par les plasmides pKan707 (piste 1), pYG1343 (piste 3) ou pYG1345 (piste 4) ; standard de poids moléculaire (piste 2).
   **SEQ ID n° 4** : Séquence nucléotidique du fragment de restriction MstII-HindIII du plasmide pYG1259 (SAH-G.CSF). La limite de la partie G-CSF (174 résidus) est indiquée. Les sites de restriction ApaI et SstI (SstI) sont soulignés. La numérotation des acides aminés correspond à la protéine chimère SAH-G.CSF mature (759 résidus).
   **SEQ ID n° 5** : Séquence nucléotidique du fragment de restriction HindIII du plasmide pYG1301 (chimère G.CSF-Gly₄-SAH). Les flèches noires indiquent la fin des régions "pré" et "pro" de la SAH. Les sites de restriction ApaI, SstI (SacI) et MstII sont soulignés. Les domaines G.CSF (174 résidus) et SAH (585 résidus) sont séparés par le linker synthétique GGGG. La numérotation des acides aminés correspond à la protéine chimère G.CSF-Gly4-SAH mature (763 résidus). La séquence nucléotidique comprise entre le codon de terminaison de la traduction et le site HindIII provient de l'ADN complémentaire (cDNA) de la SAH tel que décrit dans la demande de brevet EP 361 991.

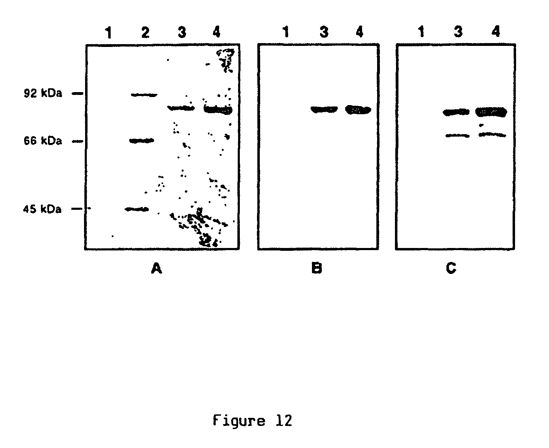

**Figure 12 :** Caractérisation du matériel sécrété après 4 jours de culture (erlenmeyers) de la souche CBS 293.91 transformée par les plasmides pYG1266 (plasmide d'expression d'une chimère du type SAH-G.CSF) et pKan707 (plasmide contrôle). Dans cette expérience les résultats des panneaux A, B, et C ont été migrés sur le même gel (SDS-PAGE 8,5 %) puis traités séparemment.
   **A,** coloration au bleu de coomassie; standard de poids moléculaire (piste 2) ; surnageant équivalent à 100 µl de la culture transformée par les plasmides pKan707 en milieu YPL (piste 1), ou pYG1266 en milieu YPD (piste 3) ou YPL (piste 4).
   **B,** caractérisation immunologique du matériel sécrété après utilisation d'anticorps primaires dirigés contre le G-CSF humain : même légende qu'en A.
   **C,** caractérisation immunologique du matériel sécrété après utilisation d'anticorps primaires dirigés contre l'albumine humaine : même légende qu'en A.

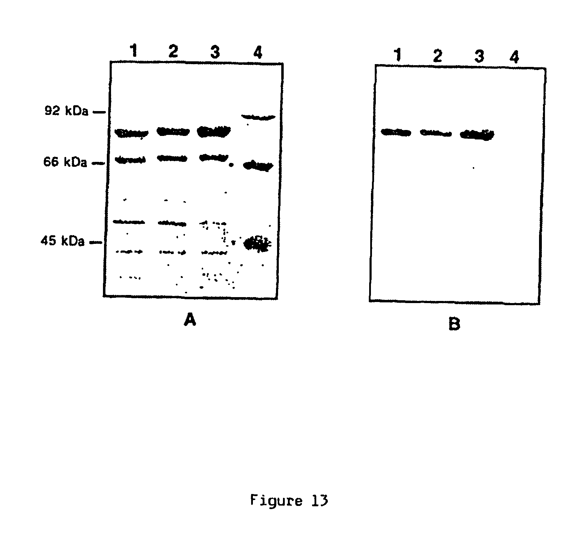

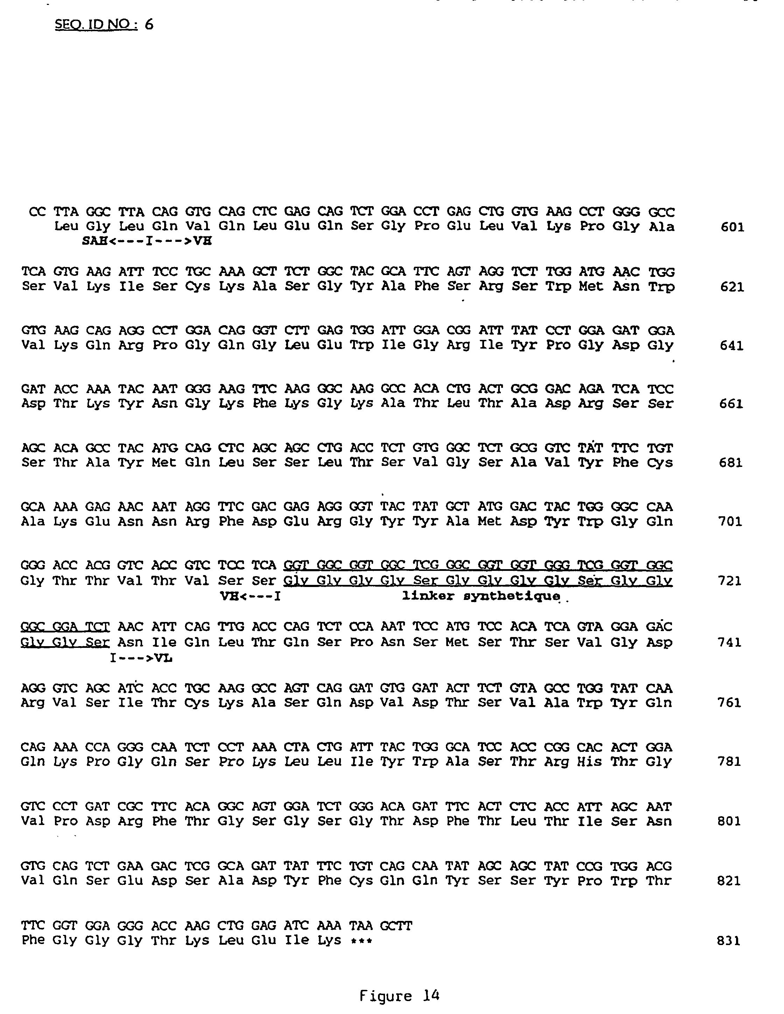

**Figure 13 :** Caractérisation du matériel sécrété après 4 jours de culture (erlenmeyers en milieu YPD) de la souche CBS 293.91 transformée par les plasmides pYG1267 (chimère SAH-G.CSF), pYG1303 (chimère G.CSF-Gly₄-SAH) et pYG1352 (chimère SAH-Gly₄-G.CSF) après migration sur gel SDS-PAGE 8,5 %.
   **A,** coloration au bleu de coomassie ; surnageant équivalent à 100 µl de la culture transformée par les plasmides pYG1303 (piste 1), pYG1267 (piste 2) ou pYG1352 (piste 3); standard de poids moléculaire (piste 4).
   **B,** caractérisation immunologique du matériel sécrété après utilisation d'anticorps primaires dirigés contre le G-CSF humain : même légende qu'en A.

   **SEQ ID n° 6 :** Séquence nucléotidique du fragment de restriction MstII-HindIII du plasmide pYG1382 (SAH-Fv'). Les domaines VH (124 résidus) et VL (107 résidus) du fragment Fv' sont séparés par le linker synthétique (GGGGS)ₓ₃. La numérotation des acides aminés correspond à la protéine chimère SAH-Fv' mature (831 résidus).

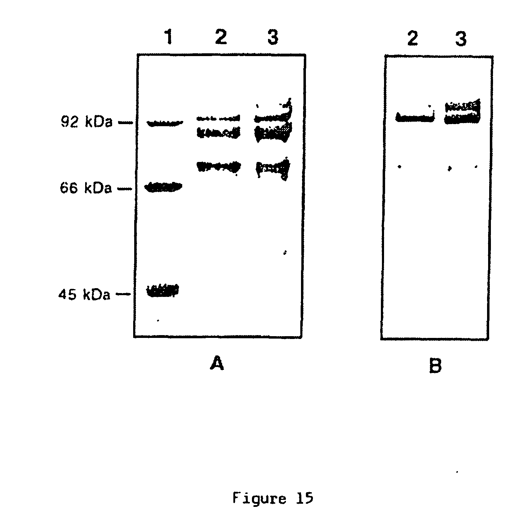

**Figure 15 :** Sécrétion de la chimère SAH-Fv' par la souche CBS 293.91 transformée par le plasmide pYG1383 (LAC4) après 4 jours de croissance en erlenmeyers à 28°C en milieu YPD (piste 2), ou YPL (piste 3) ; standard de poids moléculaire (piste 1). Les dépôts, équivalents à 200 µl de culture (précipitation à l'éthanol), sont migrés en gel PAGE-SDS (8,5 %).
   **A,** : coloration du gel au bleu de coomassie.
   **B,** : caractérisation immunologique du matériel sécrété après utilisation d'anticorps primaires dirigés contre la SAH.

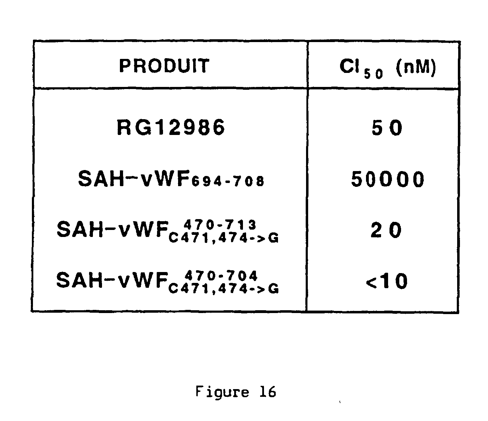

**Figure 16 :** Dosage de l'activité antagoniste in vitro de l'agglutination des plaquettes humaines fixées au formaldéhyde : CI50 des hybrides SAH-vWF694-708, [SAH-vWF470-713 C471G, C474G] et [SAH-vWF470-704 C471G, C474G] relativement à l'étalon RG12986. La détermination de l'inhibition dose-dépendante de l'agglutination plaquettaire est réalisée selon la méthode décrite par C. Prior et al. [Bio/Technology (1992) 10 66] en utilisant un agrégamètre enregistrant les variations de la transmission optique sous agitation à 37°C en présence de vWF humain, de botrocétine (8,2 mg/ml) et du produit à tester à différentes dilutions. La concentration du produit qui permet d'inhiber de moitié l'agglutination contrôle (en l'absence de produit) est alors déterminée (CI50).

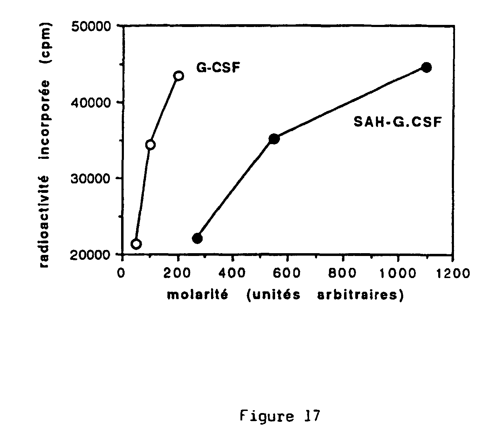

**Figure 17 :** Activité sur la prolifération cellulaire in vitro de la lignée murine NFS60. La radioactivité (³H-thymidine) incorporée dans les noyaux cellulaires après 6 heures d'incubation est représentée en ordonnée (cpm) ; la quantité de produit indiquée en abscisse est exprimée en molarité (unités arbitraires).

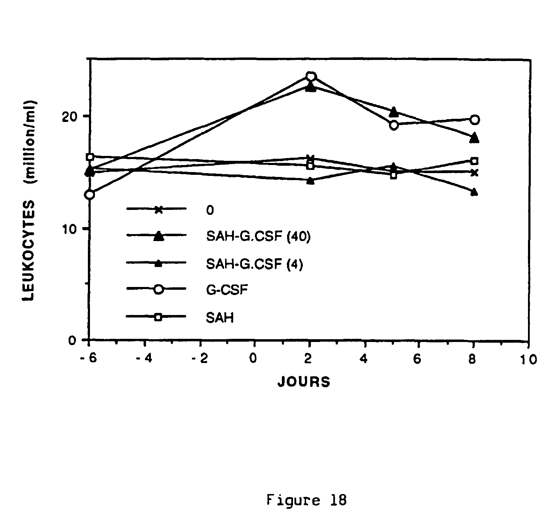

**Figure 18 :** Activité sur la granulopoièse in vivo chez le rat. Le nombre de neutrophiles (moyenne de 7 animaux) est indiquée en ordonnée en fonction du temps. Les produits testés sont la chimère SAH-G.CSF (pYG1266, 4 ou 40 mg/rat/jour), le G-CSF référence (10 mg/rat/jour), la SAH recombinante purifiée à partir de surnageant de Kluyveromyces lactis (SAH, 30 mg/rat/jour, cf. EP 361 991), ou du sérum physiologique.

### EXEMPLES

### TECHNIQUES GENERALES DE CLONAGE

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982 ; Ausubel F.M. et al. (eds), "Current Protocols in Molecular Biology", John Wiley & Sons, New York, 1987].

Les enzymes de restriction ont été fournies par New England Biolabs (Biolabs), Bethesda Research Laboratories (BRL) ou Amersham et sont utilisées selon les recommandations des fournisseurs.

Les plasmides de type pBR322, pUC et les phages de la série M13 sont d'origine commerciale (Bethesda Research Laboratories).

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes est effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques est effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] est effectuée en utilisant un "DNA thermal cycler" (Perkin Elmer Cetus) selon les spécifications du fabricant.

La vérification des séquences nucléotidiques est effectuée par la méthode développée par Sanger et al. [Proc. Natl. Acad. Sci. USA, 74 (1977) 5463-5467] en utilisant le kit distribué par Amersham.

Les transformations de K. lactis avec l'ADN des plasmides d'expression des protéines de la présente invention sont effectuées par toute technique connue de l'homme de l'art, et dont un exemple est donné dans le texte.

Sauf indication contraire, les souches bactériennes utilisées sont E. coli MC1060 (lacIPOZYA, X74, galU, galK, strA^{r}), ou E. coli TG1 (lac, proA,B, supE, thi, hsdD5 / FtraD36, proA⁺B⁺, lacI^{q}, lacZ, M15).

Les souches de levures utilisées appartiennent aux levures bourgeonnantes et plus particulièrement aux levures du genre Kluyveromyces. Les souche K. lactis MW98-8C (a, uraA, arg, lys, K⁺, pKD1°) et K. lactis CBS 293.91 ont été particulièrement utilisées; un échantillon de la souche MW98-8C a été déposé le 16 Septembre 1988 au Centraalbureau voor Schimmelkulturen (CBS) à Baarn (Pays Bas) où il a été enregistré sous le numéro CBS 579.88.

Une souche bactérienne (E. coli) transformée avec le plasmide pET-8c52K a été déposée le 17 Avril 1990 auprès de l'American Type Culture Collection sous le numéro ATCC 68306.

Les souches de levures transformées par les plasmides d'expression codant pour les protéines de la présente invention sont cultivées en erlenmeyers ou en fermenteurs pilotes de 21 (SETRIC, France) à 28°C en milieu riche (YPD: 1% yeast extract, 2% Bactopeptone, 2% glucose; ou YPL: 1% yeast extract, 2% Bactopeptone, 2% lactose) sous agitation constante.

### EXEMPLE 1 : COUPLAGE EN C-TERMINAL DE LA SAH

Le plasmide pYG404 est décrit dans la demande de brevet EP 361 991. Ce plasmide comporte un fragment de restriction HindIII codant pour le gène de la prépro-SAH précédé des 21 nucléotides naturellement présents immédiatement en amont de l'ATG initiateur de traduction du gène PGK de S. cerevisiae. La séquence nucléotidique de ce fragment de restriction est incluse dans la séquence ID n° 2. Le site MstII localisé dans la séquence codante, à trois résidus du codon spécifiant la fin de traduction est particulièrement utile comme site de clonage d'un peptide biologiquement actif que l'on désire coupler en phase traductionnelle en C-terminal de la SAH. Dans un mode de réalisation particulier, il est utile d'utiliser des peptides dont la séquence est codée par un fragment de restriction MstII-HindIII du type : 5'-CCTTAGGCTTA [3xN]p TAAGCTT-3', la séquence codant le peptide (p résidus) biologiquement actif est [3xN]p). La ligature de ce fragment avec le fragment de restriction HindIII-MstII correspondant à la totalité du gène codant pour la SAH à l'exception des trois acides aminés les plus C-terminaux (résidus leucine-glycine-leucine) génère un fragment de restriction HindIII comportant un gène hybride codant pour une protéine chimère du type SAH-PEPTIDE (Figure 1, panneau A), immédiatement précédée de la région d'exportation "prépro" de la SAH. Dans un autre mode de réalisation, le peptide biologiquement actif peut être présent plus d'une fois dans la chimère.

### EXEMPLE 2: COUPLAGE EN N-TERMINAL DE LA SAH

Dans un mode réalisation particulier, les techniques combinées de mutagénèse dirigée et d'amplification PCR permettent de construire des gènes hybrides codant pour une protéine chimère résultant du couplage traductionnel entre un peptide signal (et par exemple la région prépro de la SAH), une séquence incluant le peptide biologiquement actif et la forme mature de la SAH ou un de ses variants moléculaires. Ces gènes hybrides sont préférentiellement bordés en 5' de l'ATG initiateur de traduction et en 3' du codon de fin de traduction par des sites de restriction HindIII et codent pour des protéines chimères du type PEPTIDE-SAH (Figure 1, panneau B). Dans un mode réalisation encore plus particulier, le peptide biologiquement actif peut être présent plus d'une fois dans la chimère.

### EXEMPLE 3: COUPLAGE EN N-ET C-TERMINAL DE LA SAH

Les techniques combinées de mutagénèse dirigée et d'amplification PCR décrites dans les exemples 1 et 2 permettent de construire des gènes hybrides codant pour une protéine chimère résultant du couplage traductionnel entre la forme mature de la SAH, ou un de ses variants moléculaires, et un peptide biologiquement actif couplé aux extrémités N- et C-terminales de la SAH. Ces gènes hybrides sont préférentiellement bordés en 5' de l'ATG initiateur de traduction et en 3' du codon de fin de traduction par des sites de restriction HindIII et codent pour des protéines chimères du type PEPTIDE-SAH-PEPTIDE (Figure 1, panneau C), immédiatement précédées de la région d'exportation "prépro" de la SAH. Dans un mode réalisation encore plus particulier, le peptide biologiquement actif peut être présent plus d'une fois dans la chimère.

### EXEMPLE 4 : PLASMIDES D'EXPRESSION

Les protéines chimères des exemples précédents peuvent être exprimées dans les levures à partir de promoteurs fonctionnels, régulables ou constitutifs, tels que, par exemple, ceux présents dans les plasmides pYG105 (promoteur LAC4 de Kluyveromyces lactis), pYG106 (promoteur PGK de Saccharomyces cerevisiae), pYG536 (promoteur PHO5 de S.cerevisiae), ou des promoteur hybrides tels que ceux décrits dans la demande de brevet EP 361 991. Les plasmides pYG105 et pYG106 sont ici particulièrement utiles car ils permettent l'expression des gènes codés par les fragments de restriction HindIII tel que décrits dans les exemples précédents et clonés dans le site HindIII et dans l'orientation productive (définie comme l'orientation qui place la région "prépro" de l'albumine de façon proximale par rapport au promoteur de transcription), à partir de promoteurs fonctionnels chez K.lactis, régulables (pYG105) ou constitutifs (pYG106). Le plasmide pYG105 correspond au plasmide pKan707 décrit dans la demande de brevet EP 361 991 dans lequel le site de restriction HindIII unique et localisé dans le gène de résistance à la généticine (G418) a été détruit par mutagénèse dirigée tout en conservant une protéine inchangée (oligodeoxynucleotide 5'-GAAATGCATAAGCTCTTGCCATTCTCACCG-3'). Le fragment SalI-SacI codant pour le gène URA3 du plasmide muté a été ensuite remplacé par un fragment de restriction SalI-SacI comportant une cassette d'expression constituée du promoteur LAC4 de K. lactis (sous la forme d'un fragment SalI-HindIII) et du terminateur du gène PGK de S. cerevisiae (sous la forme d'un fragment HindIII-SacI). Le plasmide pYG105 est mitotiquement très stable chez les levures Kluyveromyces et une carte de restriction en est donnée à la Figure 3. Les plasmides pYG105 et pYG106 ne diffèrent entre eux que par la nature du promoteur de transcription encodé par le fragment SalI-HindIII.

### EXEMPLE 5 : TRANSFORMATION DES LEVURES

La transformation des levures appartenant au genre Kluyveromyces, et en particulier les souches MW98-8C et CBS 293.91 de K. lactis, s'effectue par exemple par la technique de traitement des cellules entières par de l'acétate de lithium [Ito H. et al., J. Bacteriol. 153 (1983) 163-168], adaptée comme suit. La croissance des cellules se fait à 28°C dans 50 ml de milieu YPD, avec agitation et jusqu'à une densité optique à 600 nm (DO₆₀₀) comprise entre 0,6 et 0,8; les cellules sont récoltées par centrifugation à faible vitesse, lavées dans une solution stérile de TE (10 mM Tris HCl pH 7,4; 1 mM EDTA), resuspendues dans 3-4 ml d'acétate lithium (0,1 M dans du TE) pour obtenir une densité cellulaire d'environ 2 x 10⁸ cellules/ml, puis incubées à 30°C pendant 1 heure sous agitation modérée. Des aliquotes de 0,1 ml de la suspension résultante de cellules compétentes sont incubés à 30°C pendant 1 heure en présence d'ADN et à une concentration finale de 35% de polyéthylène glycol (PEG₄₀₀₀, Sigma). Après un choc thermique de 5 minutes à 42°C, les cellules sont lavées 2 fois, resuspendues dans 0,2 ml d'eau stérile et incubées 16 heures à 28°C dans 2 ml de milieu YPD pour permettre l'expression phénotypique du gène de résistance au G418 exprimé sous contrôle du promoteur Pₖ₁ (cf. EP 361 991); 200 µl de la suspension cellulaire sont ensuite étalés sur boites YPD sélectives (G418, 200 µg/ml). Les boites sont mises à incuber à 28°C et les transformants apparaissent après 2 à 3 jours de croissance cellulaire.

### EXEMPLE 6 : SECRETION DES CHIMERES

Après sélection sur milieu riche supplémenté en G418 les clones recombinants sont testés pour leur capacité à sécréter la forme mature des protéines chimères. Quelques clones correspondant à la souche CBS 293.91 ou MW98-8C transformée par les plasmides d'expression des chimères entre la SAH et la partie biologiquement active sont mis à incuber en milieu YPD ou YPL à 28°C. Les surnageants cellulaires sont récupérés par centrifugation quand les cellules atteignent la phase stationnaire de croissance, éventuellement concentrés 10 fois par précipitation pendant 30 minutes à -20°C dans une concentration finale de 60% d'éthanol, puis testés après électrophorèse en gel SDS-PAGE à 8.5%, soit directement par coloration du gel par du bleu de coomassie, soit après immunoblot en utilisant des anticorps primaires dirigés contre la partie biologiquement active ou un sérum polyclonal de lapin dirigé contre la SAH. Lors des expériences de détection immunologique, le filtre de nitrocellulose est d'abord incubé en présence des anticorps primaires spécifiques, lavé plusieurs fois, incubé en présence d'anticorps de chèvre dirigés contre les anticorps primaires, puis incubé en présence d'un complexe avidine-péroxydase en utilisant le "kit ABC" distribué par Vectastain (Biosys S.A., Compiègne, France). La réaction immunologique est ensuite révélée par addition de diamino-3,3' benzidine tetrachlorydrate (Prolabo) en présence d'eau oxygénée, selon les recommandations du fabricant.

### EXEMPLE 7 : CHIMERES DERIVEES DU FACTEUR VON WILLEBRAND

### E.7.1. Fragments antagonistes de la fixation du vWF aux plaquettes.

### E.7.1.1. Résidus Thr470-Val713 du vWF.

Le plasmide pET-8c52K comporte un fragment du cDNA du vWF codant pour les résidus 445 à 733 du vWF humain et inclus donc plusieurs déterminants cruciaux de l'interaction entre le vWF et les plaquettes d'une part, et certains éléments de la membrane basale et du tissu sous-endothelial d'autre part, et notamment les peptides G10 et D5 antagonistes de l'interaction entre vWF et GP1b [Mori H. et al., J. Biol. Chem. 263 (1988) 17901-17904]. Cette séquence peptidique est identique à la séquence correspondante décrite par Titani et al. [Biochemistry 25 (1986) 3171-3184]. L'amplification de ces déterminants génétiques peut être réalisée à partir du plasmide pET-8c52K, par exemple par la technique d'amplification PCR, en utilisant comme amorce des oligodéoxynucléotides codant pour des résidus contigus localisés de part et d'autres de la séquence à amplifier. Les fragments amplifiés sont ensuite clonés dans des vecteurs du type M13 en vue de leur vérification par séquençage en utilisant soit les amorces universelles situées de part et d'autre du multisite de clonage, soit des oligodéoxynucléotides spécifiques de la région amplifiée du gène du vWF dont la séquence de plusieurs isomorphes est connue [Sadler J.E. et al., Proc. Natl. Acad. Sci. 82 (1985) 6394-6398; Verweij C.L. et al., EMBO J. 5 (1986) 1839-1847; Shelton-Inloes B.B. et al., Biochemistry 25 (1986) 3164-3171; Bonthron D. et al., Nucleic Acids Res. 17 (1986) 7125-7127]. Ainsi, l'amplification PCR du plasmide pET-8c52K avec les oligodéoxynucléotides 5'-CCCGGGATCCCTTAGGCTTAACCTGTGAAGCCTGC-3' (Sq1969, le site MstII est souligné) et 5'-CCCGGGATCCAAGCTTAGACTTGTGCCATGTCG-3' (Sq2029, le site HindIII est souligné) génère un fragment de restriction MstII-HindIII incluant les résidus Thr470 à Val713 du vWF (SEQ ID n° 2). La ligature de ce fragment avec le fragment de restriction HindIII-MstII correspondant à la totalité du gène codant pour la SAH à l'exception des trois acides aminés les plus C-terminaux (cf. SEQ ID n° 1) génère un fragment de restriction HindIII comportant un gène hybride codant pour une protéine chimère du type SAH-PEPTIDE (Figure 1, panneau A), immédiatement précédée de la région d'exportation "prépro" de la SAH. Ce fragment de restriction est cloné dans l'orientation productive et dans le site HindIII du plasmide pYG105, ce qui génère le plasmide d'expression pYG1248 (SAH-vWF470-713).

### E.7.1.2. Variants moléculaires.

Dans un autre mode de réalisation, le site de liaison du vWF est un peptide incluant les résidus Thr470 à Asp498 du vWF mature. Cette séquence inclus le peptide G10 (Cys474-Pro488) décrit par Mori et al. [J. Biol. Chem. 263 (1988) 17901-17904] et capable d'antagoniser l'interaction du vWF humain à la GP1b des plaquettes humaines. La séquence correspondant au peptide G10 est d'abord incluse dans un fragment de restriction MstII-HindIII (Figure 4, panneau B), par exemple par amplification PCR du plasmide pET-8c52K avec les oligodéoxynucléotides Sq1969 et 5'-CCCGGGATCCAAGCTTAGTCCTCCACATACAG-3' (Sq1970, le site HindIII est souligné), ce qui génère un fragment de restriction MstII-HindIII incluant le peptide G10, et dont la séquence est: 5'-CCTTAGGCTTAACCTGTGAAGCCTGCCAGGAGCCGGGAGGCCTGGTGGTGCCTCCCACAGATGCCCCGGTGAGCCCCACCACTCTGTATGTGGAGGACTAAGCTT-3' (la séquence codant pour le peptide G10 est en caractères gras). La ligature de ce fragment avec le fragment de restriction HindIII-MstII correspondant à la totalité du gène codant pour la SAH à l'exception des trois acides aminés les plus C-terminaux (cf. SEQ ID n° 1) génère un fragment de restriction HindIII comportant un gène hybride codant pour une protéine chimère du type SAH-PEPTIDE (Figure 1, panneau A), immédiatement précédée de la région d'exportation "prépro" de la SAH. Ce fragment de restriction est cloné dans l'orientation productive dans le site HindIII du plasmide pYG105, ce qui génère le plasmide d'expression pYG1214.

Dans un autre mode de réalisation, le site de liaison du vWF à la GP1b est directement conçu à l'aide d'oligodéoxynucléotides synthétiques, et par exemple les oligodéoxynucléotides 5'-TTAGGCCTCTGTGACCTTGCCCCTGAAGCCCCTCCTCCTACTCTGCCCCCCTAAGCTTA-3' et 5'-GATCTAAGCTTAGGGGGGCAGAGTAGGAGGAGGGGCTTCAGGGGCAAGGTCACAGAGGCC-3'. Ces oligodéoxynucléotides forment en s'appariant un fragment de restriction MstII-BglII incluant le fragment MstII-HindIII (Figure 4, panneau C) correspondant au peptide D5 défini par les résidus Leu694 à Pro708 du vWF. La ligature du fragment MstII-HindIII avec le fragment de restriction HindIII-MstII correspondant à la totalité du gène codant pour la SAH à l'exception des trois acides aminés les plus C-terminaux (cf. SEQ ID n° 1) génère un fragment de restriction HindIII comportant un gène hybride codant pour une protéine chimère du type SAH-PEPTIDE (Figure 1, panneau A), immédiatement précédée de la région d'exportation "prépro" de la SAH. Ce fragment de restriction est cloné dans l'orientation productive dans le site HindIII du plasmide pYG105, ce qui génère le plasmide d'expression pYG1206.

Des variants utiles du plasmide pET-8c52K sont délétés par mutagénèse dirigée entre les peptides G10 et D5, par exemple des sites de fixation au collagène, et/ou à l'héparine, et/ou à la botrocétine, et/ou aux sulfatides et/ou à la ristocétine. Un exemple est le plasmide pMMB9 délété par mutagénèse dirigée entre les résidus Cys509 et Ile662. L'amplification PCR de ce plasmide avec les oligodéoxynucléotides Sq1969 et Sq2029 génère un fragment de restriction MstII-HindIII (Figure 4, panneau D) incluant les résidus Thr470 à Tyr508 et Arg663 à Val713 et en particulier les peptides G10 et D5 du vWF et délété en particulier de son site de fixation au collagène localisé entre les résidus Glu542 et Met622 [Roth GJ. et al. Biochemistry 25 (1986) 8357-8361]. La ligature de ce fragment avec le fragment de restriction HindIII-MstII correspondant à la totalité du gène codant pour la SAH à l'exception des trois acides aminés les plus C-terminaux (cf. SEQ ID n° 1) génère un fragment de restriction HindIII comportant un gène hybride codant pour une protéine chimère du type SAH-PEPTIDE (Figure 1, panneau A), immédiatement précédée de la région d'exportation "prépro" de la SAH. Ce fragment de restriction est cloné dans l'orientation productive dans le site HindIII du plasmide pYG105, ce qui génère le plasmide d'expression pYG1223.

Dans d'autres modes de réalisation, l'utilisation des techniques combinées de mutagénèse dirigée et d'amplification PCR permet de générer à volonté des variants du fragment de restriction MstII-HindIII du panneau A de la Figure 4 mais délétés d'un ou plusieurs sites de fixation aux sulfatides et/ou à la botrocétine et/ou à l'héparine et/ou au collagène, et/ou substitué par tout résidu impliqué dans l'émergence de pathologies de type IIB associée au vWF.

Dans d'autres variants utiles du plasmide pET-8c52K des mutations sont introduites, par exemple par mutagénèse dirigée, pour remplacer ou supprimer tout ou partie de l'ensemble des cystéines présentes aux positions 471, 474, 509 et 695 du vWF humain. Des exemples particuliers sont les plasmides p5E et p7E dans lesquels les cystéines présentes aux positions 471 et 474 d'une part et aux positions 471, 474, 509 et 695 d'autre part ont été respectivement remplacés par des résidus glycine. L'amplification PCR de ces plasmides avec les oligodéoxynucléotides Sq2149 (5'-CCCGGGATCCCTTAGGCTTAACCGGTGAAGCCGGC-3', le site MstII est souligné) et Sq2029 permet de générer des fragments de restriction MstII-HindIII incluant les résidus Thr470 à Val713 du vWF naturel à l'exception qu'au moins les résidus cystéine aux positions 471 et 474 ont été mutés en des résidus glycine. La ligature de ces fragments avec le fragment de restriction HindIII-MstII correspondant à la totalité du gène codant pour la SAH à l'exception des trois acides aminés les plus C-terminaux (cf. SEQ ID n° 1) génère un fragment de restriction HindIII comportant un gène hybride codant pour une protéine chimère du type SAH-PEPTIDE (Figure 1, panneau A), immédiatement précédée de la région d'exportation "prépro" de la SAH. Ces fragments de restriction sont clonés dans l'orientation productive dans le site HindIII du plasmide pYG105, ce qui génère les plasmides d'expression pYG1283 (chimère SAH-vWF470-713, C471G, C474G) et pYG1279 (chimère SAH-vWF470-713, C471G, C474G, C509G, C695G).

D'autres mutations particulièrement utiles concernent au moins un résidu impliqué dans des pathologies de type IIB associées au vWF (augmentation de l'affinité intrinsèque du vWF pour la GP1b), comme les résidus Arg543, Arg545, Trp550, Val551, Val553, Pro574 ou Arg578 par exemple. Les techniques de recombinaison génétique in vitro permettent également d'introduire à volonté un ou des résidus supplémentaires dans la séquence du vWF et par exemple une méthionine surnuméraire entre les positions Asp539 et Glu542.

### E.7.2. Fragments antagonistes de la fixation du vWF au sous endothélium.

Dans un mode de réalisation particulier, les sites de liaison du vWF aux composants du tissu sous-endothélial, et par exemple du collagène, sont générés par amplication PCR du plasmide pET-8c52K, par exemple avec les oligodéoxynucléotides Sq2258 (5'-GGATCCTTAGGGCTG-TGCAGCAGGCTACTGGACCTGGTC-3', le site MstII est souligné) et Sq2259 (5'-GAATTCAAGCTTAACAGAGGTAGCTAACGATCTCGTCCC-3', le site HindIII est souligné), ce qui génère un fragment de restriction MstII-HindIII codant pour les résidus Cys509 à Cys695 du vWF naturel. Des variants moléculaires de délétion ou modifiés sont également générés qui comportent toute combinaison souhaitable entre les sites de fixation du vWF aux sulfatides et/ou à la botrocétine et/ou à l'héparine et/ou au collagène et/ou tout résidu responsable d'une modification de l'affinité du vWF pour la GP1b (pathologies de type II associée au vWF). Dans un autre mode de réalisation, le domaine capable de se fixer au collagène peut également provenir du fragment du vWF compris entre les résidus 911 et 1114 et décrit par Pareti et al. [J. Biol. Chem. (1987) 262: 13835-13841]. La ligature de ces fragments avec le fragment de restriction HindIII-MstII correspondant à la totalité du gène codant pour la SAH à l'exception des trois acides aminés les plus C-terminaux (cf. SEQ ID n° 1) génère des fragments de restriction HindIII comportant un gène hybride codant pour une protéine chimère du type SAH-PEPTIDE (Figure 1, panneau A), immédiatement précédée de la région d'exportation "prépro" de la SAH. Ces fragments de restriction sont clonés dans l'orientation productive dans le site HindIII du plasmide pYG105, ce qui génère les plasmides d'expression correspondants, et par exemple le plasmide pYG1277 (SAH-vWF509-695).

### E.7.3. Purification et caractérisation moléculaire des chimères entre SAH et vWF.

Les chimères présentes dans les surnageants de culture correspondant à la souche CBS 293.91 transformée, par exemple par les plasmides d'expression selon les exemples E.7.1. et E.7.2., sont caractérisées dans un premier temps à l'aide d'anticorps spécifiques de la partie SAH et de la partie vWF. Les résultats des Figures 5 à 7 démontrent que la levure K. lactis est capable de sécréter des protéines chimères entre la SAH et un fragment du vWF, et que ces chimères sont immunologiquement réactives. Il peut être également souhaitable de purifier certaines de ces chimères. La culture est alors centrifugée (10000 g, 30 min), le surnageant est passé à travers un filtre de 0,22 mm (Millipore), puis concentré par ultrafiltration (Amicon) en utilisant une membrane dont le seuil de discrimination se situe à 30 kDa. Le concentrat obtenu est alors dialysé contre une solution de Tris HCl (50 mM pH 8) puis purifié sur colonne. Par exemple, le concentrat correspondant au surnageant de culture de la souche CBS 293.91 transformée par le plasmide pYG1206 est purifiée par chromatographie d'affinité sur Bleu-Trisacryl (IBF). Une purification par chromatographie d'échange d'ions peut également être utilisée. Par exemple dans le cas de la chimère SAH-vWF470-713, le concentrat obtenu après ultrafiltration est dialysé contre une solution de Tris HCl (50 mM pH 8), puis déposé par fractions de 20 ml sur une colonne (5 ml) échangeuse de cations (S Fast Flow, Pharmacia) équilibrée dans le même tampon. La colonne est alors lavée plusieurs fois par la solution de Tris HCl (50 mM pH 8) et la protéine chimère est alors éluée de la colonne par un gradient (0 à 1 M) de NaCl. Les fractions contenant la protéine chimère sont alors réunies et dialysées contre une solution de Tris HCl 50 mM (pH 8) puis redéposées sur colonne S Fast Flow. Après élution de la colonne, les fractions contenant la protéine sont réunies, dialysées contre de l'eau et lyophilisées avant caractérisation: par exemple, le séquençage (Applied Biosystem) de la protéine [SAH-vWF470-704 C471G, C474G] sécrétée par la levure CBS 293.91 donne la séquence N-terminale attendue de la SAH (Asp-Ala-His...), démontrant une maturation correcte de la chimère immédiatement en C-terminal du doublet de résidus Arg-Arg de la région "pro" de la SAH (SEQ ID n° 1). Le caractère essentiellement monomérique des protéines chimères entre SAH et vWF est également confirmé par leur profil d'élution sur colonne TSK 3000 [Toyo Soda Company, équilibrée par une solution de cacodylate (pH 7) contenant 0,2 M de Na₂SO₄] : par exemple la chimère [SAH-vWF 470-704 C471G, C474G] se comporte dans ces conditions comme une protéine de poids moléculaire apparent de 95 kDa démontrant son caractère monomérique.

### EXEMPLE 8 : CHIMERES DERIVEES DE L'UROKINASE

### E.8.1. Constructions.

Un fragment correspondant au fragment amino-terminal de l'urokinase (ATF: domaine EGF-like + domaine kringle) peut être obtenu à partir de l'ARN messager correspondant des cellules de certains carcinome humain, par exemple en utilisant le kit RT-PCR distribué par Pharmacia. Un fragment de restriction MstII-HindIII incluant l'ATF de l'urokinase humaine est donné SEQ ID n° 3. La ligature du fragment HindIII-MstII du plasmide pYG404 avec ce fragment MstII-HindIII permet de générer le fragment HindIII du plasmide pYG1341 qui code pour une protéine chimère dans laquelle la molécule de SAH est génétiquement couplée à l'ATF (SAH-UK1->135). De façon similaire, le plasmide pYG1340 contient un fragment HindIII codant pour une chimère composée de la SAH immédiatement suivi par les 46 premiers résidus de l'urokinase humaine (SAH-UK1->46, cf. SEQ ID n° 3). Le clonage dans l'orientation productive du fragment de restriction HindIII du plasmide pYG1340 (SAH-UK1->46) dans le site HindIII des plasmides pYG105 (LAC4) et pYG106 (PGK) génère les plasmides d'expression pYG1343 et pYG1342, respectivement. De façon similaire, le clonage dans l'orientation productive du fragment de restriction HindIII du plasmide pYG1341 (SAH-UK1->135) dans le site HindIII des plasmides pYG105 (LAC4) et pYG106 (PGK) génère les plasmides d'expression pYG1345 et pYG1344, respectivement.

### E-8.2. Sécrétion des hybrides.

Après sélection sur milieu riche supplémenté en G418 les clones recombinants sont testés pour leur capacité à sécréter la forme mature des protéines chimères SAH-UK. Quelques clones correspondant à la souche K. lactis CBS 293.91 transformée par les plasmides d'expression selon l'exemple E.9.1. sont mis à incuber en milieu liquide complet sélectif à 28°C. Les surnageants cellulaires sont alors testés après électrophorèse en gel d'acrylamide à 8.5 %, soit directement par coloration du gel au bleu de coomassie, soit après immunoblot en utilisant comme anticorps primaires un sérum polyclonal de lapin dirigé contre l'albumine humaine ou contre l'urokinase humaine. Les résultats de la Figure 9 démontrent que les protéines hybrides SAH-UK1->46 et SAH-UK1->135 sont particulièrement bien sécrétées par la levure Kluyveromyces.

### E.8.3. Purification des chimères entre SAH et urokinase.

Après centrifugation d'une culture de la souche CBS 293.91 transformée par les plasmides d'expression selon l'exemple E.8.1., le surnageant de culture est passé à travers un filtre de 0,22 mm (Millipore), puis concentré par ultrafiltration (Amicon) en utilisant une membrane dont le seuil de discrimination se situe à 30 kDa. Le concentrat obtenu est alors ajusté à 50 mM Tris HCl à partir d'une solution stock de Tris HCl 1M (pH 7), puis déposé par fractions de 20 ml sur une colonne (3 ml) échangeuse d'anions (D-Zephyr, Sepracor) équilibrée dans le même tampon. La protéine chimère (SAH-UK1->46 ou SAH-UK1->135) est alors éluée de la colonne par un gradient (0 à 1 M) de NaCl. Les fractions contenant la protéine chimère sont alors réunies et dialysées contre une solution de Tris HCl 50 mM (pH 6) et redéposées sur colonne D-Zephyr équilibrée dans le même tampon. Après élution de la colonne, les fractions contenant la protéine sont réunies, dialysées contre de l'eau et lyophilisées avant caractérisation de leur activité biologique et notamment vis à vis de leur aptitude à déplacer l'urokinase de son récepteur cellulaire.

### EXEMPLE 9 : CHIMERES DERIVEES DU G-CSF

### E.9.1. Constructions.

### E.9.1.1. Couplage en C-terminal de la SAH.

Un fragment de restriction MstII-HindIII incluant la forme mature du G-CSF humain est généré, par exemple selon la stratégie suivante : un fragment de restriction KpnI-HindIII est d'abord obtenu par la technique d'amplification enzymatique PCR en utilisant les oligodéoxynucléotides Sq2291 (5'-CAAGGATCCAAGCTTCAGGGCTGCGCAAGGTGGCGTAG-3', le site HindIII est souligné) et Sq2292 (5'-CGGGGTACCTTAGGCTTAACCCCCCTGGGCCCTGCCAGC-3', le site KpnI est souligné) comme amorce sur le plasmide BBG13 servant comme matrice. Le plasmide BBG13 comporte le gène codant pour la forme B (174 acides aminés) du G-CSF mature humain, obtenu auprès de British Bio-technology Limited, Oxford, England. Le produit d'amplification enzymatique d'environ 550 nucléotides est ensuite digéré par les enzymes de restriction KpnI et HindIII et cloné dans le vecteur pUC19 coupé par les mêmes enzymes, ce qui génère le plasmide recombinant pYG1255. Ce plasmide est la source d'un fragment de restriction MstII-HindIII permettant de fusionner le G-CSF immédiatement en aval de la SAH (chimère SAH-G.CSF) et dont la séquence nucléotidique est donnée SEQ ID n° 4.

Il peut être également souhaitable d'insérer un linker peptidique entre la partie SAH et G-CSF, par exemple pour permettre une meilleure présentation fonctionnelle de la partie transductrice. Un fragment de restriction MstII-HindIII est par exemple généré par substitution du fragment MstII-ApaI du plasmide pYG1255 par les oligodéoxynucléotides Sq2742 (5'-TTAGGCTTAGGTGGTGGCGGTACCCCCCTGGGCC-3', les codons codant pour les résidus glycine de ce linker particulier sont soulignés) et Sq2741 (5'-CAGGGGGGTACCGCCACCACCTAAGCC-3') qui forment en s'appariant un fragment MstII-ApaI. Le plasmide ainsi généré comporte donc un fragment de restriction MstII-HindIII, dont la séquence est identique à celle de la SEQ ID n° 4 à l'exception du fragment MstII-ApaI.

La ligature du fragment HindIII-MstII du plasmide pYG404 avec le fragment MstII-HindIII du plasmide pYG1255 permet de générer le fragment HindIII du plasmide pYG1259 qui code pour une protéine chimère dans laquelle la forme B du G-CSF mature est positionnée par couplage génétique en phase traductionnelle en C-terminal de la molécule de SAH (SAH-G.CSF).

Un fragment de restriction HindIII identique à l'exception du fragment MstII-ApaI peut également être facilement généré et qui code pour une protéine chimère dans laquelle la forme B du G-CSF mature est positionnée par couplage génétique en phase traductionnelle en C-terminal de la molécule de SAH et d'un linker peptidique particulier. Par exemple ce linker est constitué de 4 résidus glycine dans le fragment HindIII du plasmide pYG1336 (chimère SAH-Gly₄-G.CSF).

Le fragment de restriction HindIII du plasmide pYG1259 est cloné dans l'orientation productive et dans le site de restriction HindIII du plasmide d'expression pYG105, ce qui génère le plasmide d'expression pYG1266 (SAH-G.CSF). Dans une autre exemplification, le clonage du fragment de restriction HindIII du plasmide pYG1259 dans l'orientation productive et dans le site HindIII du plasmide pYG106 génère le plasmide pYG1267. Les plasmides pYG1266 et pYG1267 sont isogéniques entre eux à l'exception du fragment de restriction SalI-HindIII codant pour le promoteur LAC4 de K. lactis (plasmide pYG1266) ou le promoteur PGK de S. cerevisiae (plasmide pYG1267).

Dans une autre exemplification, le clonage dans l'orientation productive du fragment de restriction HindIII du plasmide pYG1336 (chimère SAH-Gly₄-G.CSF) dans le site HindIII des plasmides pYG105 (LAC4) et pYG106 (PGK) génère les plasmides d'expression pYG1351 et pYG1352, respectivement.

### E.9.1.2. Couplage en N-terminal de la SAH.

Dans un mode réalisation particulier, les techniques combinées de mutagénèse dirigée et d'amplification PCR permettent de construire des gènes hybrides codant pour une protéine chimère résultant du couplage traductionnel entre un peptide signal (et par exemple la région prépro de la SAH), une séquence incluant un gène ayant une activité G-CSF, et la forme mature de la SAH ou un de ses variants moléculaires (cf. chimère du panneau B, Figure 1). Ces gènes hybrides sont préférentiellement bordés en 5' de l'ATG initiateur de traduction et en 3' du codon de fin de traduction par des sites de restriction HindIII. Par exemple l'oligodéoxynucléotide Sq2369 (5'-GTTCTACGCCACCTTGCGCAGCCCGGTGGAGGCGGTGATGCACACAAGAGTGAGGTTGCTCATCGG-3', les résidus soulignés (optionnels) correspondent dans cette chimère particulière à un linker peptidique composé de 4 résidus glycine) permet par mutagénèse dirigée de mettre en phase traductionelle la forme mature du G-CSF humain du plasmide BBG13 immédiatement en amont de la forme mature de la SAH, ce qui génère le plasmide intermédiaire A. De façon similaire, l'utilisation de l'oligodéoxynucléotide Sq2338 [5'-CAGGGAGCTGGCAGGGCCCAGGGGGGTTCGACGAAACACACCCCTGGAATAAGCCGAGCT-3' (brin non codant), les nucléotides complémentaires aux nucléotides codant pour les premiers résidus N-terminaux de la forme mature du G-CSF humain sont soulignés] permet par mutagénèse dirigée de coupler en phase traductionnelle de lecture la région prépro de la SAH immédiatement en amont de la forme mature du G-CSF humain, ce qui génère le plasmide intermédiaire B. On génère ensuite un fragment HindIII codant pour une protéine chimère du type PEPTIDE-SAH (cf. Figure 1, panneau B) en associant le fragment HindIII-SstI du plasmide B (jonction région prépro de la SAH + fragment N-terminal du G-CSF mature) avec le fragment SstI-HindIII du plasmide A [jonction G-CSF mature-(glycine)ₓ₄-SAH mature]. Le plasmide pYG1301 contient ce fragment de restriction HindIII particulier codant pour la chimère G.CSF-Gly₄-SAH fusionnée immédiatement en aval de la région prépro de la SAH (SEQ ID n° 5). Le clonage de ce fragment de restriction HindIII dans l'orientation productive et dans le site HindIII des plasmides pYG105 (LAC4) et pYG106 (PGK) génère les plasmides d'expression pYG1302 et pYG1303, respectivement.

### E.9.2. Sécrétion des hybrides.

Après sélection sur milieu riche supplémenté en G418 les clones recombinants sont testés pour leur capacité à sécréter la forme mature des protéines chimères entre SAH et G-CSF. Quelques clones correspondant à la souche K. lactis CBS 293.91 transformée par les plasmides pYG1266 ou pYG1267 (SAH-G.CSF), pYG1302 ou pYG1303 (G.CSF-Gly₄-SAH) ou encore pYG1351 ou pYG1352 (SAH-Gly₄-G.CSF) sont mis à incuber en milieu liquide complet sélectif à 28°C. Les surnageants cellulaires sont alors testés après électrophorèse en gel d'acrylamide à 8.5 %, soit directement par coloration du gel au bleu de coomassie, soit après immunoblot en utilisant comme anticorps primaires des anticorps polyclonaux de lapin dirigés contre le G-CSF humain ou un sérum polyclonal de lapin dirigé contre l'albumine humaine. Les résultats de la Figure 12 démontrent que la protéine hybride SAH-G.CSF est reconnue à la fois par des anticorps dirigés contre l'albumine humaine (panneau C) et le G-CSF humain (panneau B). Les résultats de la Figure 13 indiquent que la chimère SAH-Gly₄-G.CSF (piste 3) est particulièrement bien sécrétée par la levure Kluyveromyces, possiblement du fait que la présence du linker peptidique entre partie SAH et partie G-CSF est plus favorable à un repliement indépendant de ces 2 parties lors du transit de la chimère dans la voie sécrétoire. De plus la fusion N-terminale (G.CSF-Gly₄-SAH) est également sécrétée par la levure Kluyveromyces (Figure 13, piste 1).

### E.9.3. Purification et caractérisation moléculaire des chimères entre SAH et G-CSF.

Après centrifugation d'une culture de la souche CBS 293.91 transformée par les plasmides d'expression selon l'exemple E.9.1., le surnageant de culture est passé à travers un filtre de 0,22 mm (Millipore), puis concentré par ultrafiltration (Amicon) en utilisant une membrane dont le seuil de discrimination se situe à 30 kDa. Le concentrat obtenu est alors ajusté à 50 mM Tris HCl à partir d'une solution stock de Tris HCl 1M (pH 6), puis déposé par fractions de 20 ml sur une colonne (5 ml) échangeuse d'ions (Q Fast Flow, Pharmacia) équilibrée dans le même tampon. La protéine chimère est alors éluée de la colonne par un gradient (0 à 1 M) de NaCl. Les fractions contenant la protéine chimère sont alors réunies et dialysées contre une solution de Tris HCl-50 mM (pH 6) et redéposées sur colonne Q Fast Flow (1 ml) équilibrée dans le même tampon. Après élution de la colonne, les fractions contenant la protéine sont réunies, dialysées contre de l'eau et lyophilisées avant caractérisation: par exemple, le séquençage (Applied Biosystem) de la protéine SAH-G.CSF sécrétée par la levure CBS 293.91 donne la séquence N-terminale attendue de la SAH (Asp-Ala-His...), démontrant une maturation correcte de la chimère immédiatement en C-terminal du doublet de résidus Arg-Arg de la région "pro" de la SAH (SEQ ID n° 1).

### EXEMPLE 10 : CHIMERES DERIVEES D'UNE IMMUNOGLOBULINE

### E.10.1. Constructions.

Un fragment Fv' peut être construit par les techniques du génie génétique, et qui code pour les fragments variables des chaines lourdes et légères d'une immunoglobuline (Ig), reliés entre eux par un peptide linker [Bird et al., Science (1988) 242: 423 ; Huston et al., (1988) Proc. Natl. Acad. Sci. 85: 5879]. Schématiquement, les régions variables (environ 120 résidus) des chaines lourdes et légères d'une Ig donnée sont clonées à partir de l'ARN messager de l'hybridome correspondant, par exemple en utilisant le kit RT-PCR distribué, par Pharmacia (Mouse ScFv Module). Dans une seconde étape les régions variables sont génétiquement couplées par génie génétique par l'intermédiaire d'un peptide de liaison synthétique et par exemple le linker (GGGGS)ₓ₃. Un fragment de restriction MstII-HindIII incluant le fragment Fv' d'une immunoglobuline sécrétée par un hybridome murin est donné SEQ ID n° 6. La ligature du fragment HindIII-MstII du plasmide pYG404 avec ce fragment MstII-HindIII permet de générer le fragment HindIII du plasmide pYG1382 qui code pour une protéine chimère dans laquelle la molécule de SAH est génétiquement couplée au fragment Fv' de la SEQ ID n° 6 (chimère SAH-Fv'). Le clonage dans l'orientation productive du fragment de restriction HindIII du plasmide pYG1382 dans le site HindIII des plasmides pYG105 (LAC4) et pYG106 (PGK) génère les plasmides d'expression pYG1383 et pYG1384, respectivement.

### E.10.2. Sécrétion des hybrides.

Après sélection sur milieu riche supplémenté en G418 les clones recombinants sont testés pour leur capacité à sécréter la forme mature de la protéine chimère SAH-Fv'. Quelques clones correspondant à la souche K. lactis CBS 293.91 transformée par les plasmides pYG1383 ou pYG1384 (SAH-Fv') sont mis à incuber en milieu liquide complet sélectif à 28°C. Les surnageants cellulaires sont alors testés après électrophorèse en gel d'acrylamide à 8.5 %, soit directement par coloration du gel au bleu de coomassie, soit après immunoblot en utilisant comme anticorps primaires un sérum polyclonal de lapin dirigé contre l'albumine humaine, ou directement incubée avec des anticorps biotinylés et dirigés contre les immunoglobulines d'origine murine. Les résultats de la Figure 15 démontrent que la protéine hybride SAH-Fv' est reconnue à la fois par des anticorps dirigés contre l'albumine humaine (panneau C) et réagit avec des anticorps de chèvre biotinylés immunologiquement réactifs à l'encontre d'immunoglobulines de souris (panneau B).

### EXEMPLE 11 : ACTIVITE BIOLOGIQUE DES CHIMERES

### E.11.1. Activité biologique in vitro.

### E.11.1.1. Chimères entre SAH et vWF.

L'activité antagoniste des produits est déterminée par mesure de l'inhibition dose-dépendante de l'agglutination des plaquettes humaines fixées au paraformaldéhyde selon la méthode décrite par Prior et al. [Bio/Technology (1992) 10: 66]. Les mesures se font dans un agrégamètre (PAP-4, Bio Data, Horsham, PA, USA) qui enregistre les variations au cours du temps de la transmission optique sous agitation à 37°C en présence de vWF, de botrocétine (8,2 mg/ml) et du produit à tester à différentes dilutions (concentrations). Pour chaque mesure, 400 ml (8x10⁷ plaquettes) d'une suspension de plaquettes humaines stabilisées au paraformaldéhyde (0,5 %, puis resuspendues en [NaCl (137 mM) ; MgCl₂ (1 mM) ; NaH₂PO₄ (0,36 mM) ; NaHCO₃ (10 mM) ; KCl (2,7 mM) ; glucose (5,6 mM) ; SAH (3,5 mg/ml) ; tampon HEPES (10 mM, pH 7,35)] sont préincubés à 37°C dans la cuve cylindrique (8,75 x 50 mm, Wellcome Distriwell, 159 rue Nationale, Paris) de l'agrégamètre pendant 4 min puis sont additionnés de 30 ml de la solution du produit à tester à différentes dilutions dans du véhicule de formulation apyrogène [mannitol (50 g/l) ; acide citrique (192 mg/l) ; L-lysine monochlorhydratée (182,6 mg/l) ; NaCl (88 mg/l) ; pH ajusté à 3,5 par addition de NaOH (1M)], ou de véhicule de formulation uniquement (essai contrôle). La suspension résultante est alors incubée pendant 1 min à 37°C et on ajoute 12,5 ml de vWF humain [American Bioproducts, Parsippany, NJ, USA; 11 % d'activité von Willebrand mesurée selon les recommandations d'utilisation du PAP-4 (Platelet Aggregation Profiler^{R}) à l'aide de plaquettes fixées au formaldéhyde (2x10⁵ plaquettes/ml), de plasma humain contenant de 0 à 100 % de vWF et de ristocétine (10 mg/ml, cf. p. 36-45 : vW Program^{TM}] que l'on incube à 37°C pendant 1 min avant d'ajouter 12,5 ml de la solution de botrocétine [purifiée à partir de venin lyophilisé de Bothrops jararaca (Sigma), selon le protocole décrit par Sugimoto et al., Biochemistry (1991) 266: 18172]. L'enregistrement de la lecture de la transmission en fonction du temps est alors réalisée pendant 2 min sous agitation à l'aide d'un barreau aimanté (Wellcome Distriwell) placé dans la cuve et sous une agitation magnétique de 1100 tr/min assurée par l'agrégamètre. La variation moyenne de la transmission optique (n³5 pour chaque dilution) au cours du temps est donc une mesure de l'agglutination plaquettaire due à la présence de vWF et de botrocétine, en l'absence ou en présence de concentrations variables du produit à tester. A partir de tels enregistrements, on détermine alors le % d'inhibition de l'agglutination plaquettaire due à chaque concentration de produit et on trace la droite donnant le % d'inhibition en fonction de l'inverse de la dilution de produit en échelle log-log. La CI50 (ou concentration de produit provoquant 50 % d'inhibition de l'agglutination) est alors déterminée sur cette droite. Le Tableau de la Figure 16 compare les CI50 de quelques unes des chimères SAH-vWF de la présente invention et démontre que certaines d'entre elles sont de meilleurs antagonistes de l'agglutination plaquettaire que le produit RG 12986 décrit par Prior et al. [Bio/Technology (1992) 10: 66] et inclus dans les essais à titre de valeur étalon. Des tests identiques de l'inhibition de l'agglutination de plaquettes humaines en présence de vWF de plasma de porc (Sigma) permet en plus de démontrer que certains des hybrides de la présente invention, et notamment certains variants de type IIB, sont de très bons antagonistes de l'agglutination plaquettaire en l'absence de co-facteurs de type botrocétine. L'antagonisme botrocétine-indépendant de ces chimères particulières peut également être démontré selon le protocole initialement décrit par Ware et al. [Proc. Natl. Acad. Sci. (1991) 88: 2946] par déplacement de l'anticorps monoclonal ¹²⁵I-LJ-IB1 (10 mg/ml), un inhibiteur compétitif de la fixation du vWF sur la GPIb plaquettaire [Handa M. et al., (1986) J. Biol. Chem. 261: 12579] après 30 min d'incubation à 22°C en présence de plaquettes fraiches (10⁸ plaquettes/ml).

### E.11.1.2. Chimères entre SAH et G-CSF.

Les chimères purifiées sont testées pour leur capacité à permettre la prolifération in vitro de la lignée murine IL3-dépendante NFS60, par mesure de l'incorporation de thymidine tritiée essentiellement selon le protocole décrit par Tsuchiya et al. (Proc. Natl. Acad. Sci. (1986) 83 7633]. Pour chaque chimère, les mesures sont réalisées entre 3 et 6 fois dans un test trois points (trois dilutions du produit) dans une zone ou la relation entre quantité de produit actif et incorporation de thymidine marquée (Amersham) est linéaire. Dans chaque plaque de microtitration, l'activité d'un produit référence constitué de G-CSF humain recombinant exprimé dans des cellules mammifères est également systématiquement incorporé. Les résultats de la Figure 17 démontrent que la chimère SAH-G.CSF (pYG1266) sécrétée par la levure Kluyveromyces et purifiée selon l'exemple E.9.3. est capable in vitro de transduire un signal de prolifération cellulaire pour la lignée NFS60. Dans ce cas particulier, l'activité spécifique (cpm/molarité) de la chimère est environ 7 fois plus faible que celle du G-CSF référence (non couplé).

### E.11.2. Activité biologique in vivo.

L'activité de stimulation des chimères SAH/G-CSF sur la granulopoièse in vivo est testée après injection sous-cutanée chez le rat (Sprague-Dawley/CD, 250-300 g, 8-9 semaines) et comparée à celle du G-CSF référence exprimé à partir de cellules de mammifère. Chaque produit, testé à raison de 7 animaux, est injecté par voie sous-cutanée en région dorso-scapulaire à raison de 100 ml pendant 7 jours consécutifs (J1-J7). 500 ml de sang sont receuillis aux jours J-6, J2 (avant la 2^{ème} injection), J5 (avant la 5^{ème} injection) et J8, et une numération sanguine est effectuée. Dans ce test, l'activité spécifique (unités de neutropoièse/mole injectée) de la chimère SAH-G.CSF (pYG1266) est identique à celle du G-CSF référence (Figure 18). Puisque cette chimère particulière possède in vitro une activité spécifique 7 fois plus faible que celle du G-CSF référence (Figure 17), il est donc démontré que le couplage génétique du G-CSF sur la SAH en modifie favorablement les propriétés pharmacocinétiques.

### LISTE DE SEQUENCES

(2) INFORMATION POUR LA SEQ ID NO: 1:
   (i) CARACTERISTIQUES DE LA SEQUENCE:
      (A) LONGUEUR: 1859 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 26..1855
      (D) AUTRES RENSEIGNEMENTS: /product= "Chimere de type SAH-Peptide"
   (ix) CARACTERISTIQUE ADDITIONELLE:
      (A) NOM/CLE: misc_feature
      (B) EMPLACEMENT: 1842..1848
      (D) AUTRES RENSEIGNEMENTS: /standard_name= "Site Mst II"
(2) INFORMATION POUR LA SEQ ID NO: 2:
   (i) CARACTERISTIQUES DE LA SEQUENCE:
      (A) LONGUEUR: 750 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 3..746
      (D) AUTRES RENSEIGNEMENTS: /product= "Fragment C-ter de la chimere SAH-vWF470"
(2) INFORMATION POUR LA SEQ ID NO: 3:
   (i) CARACTERISTIQUES DE LA SEQUENCE:
      (A) LONGUEUR: 423 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 3..419
      (D) AUTRES RENSEIGNEMENTS: /product= "Fragment C-ter de la chimere SAH-UK1-135"
(2) INFORMATION POUR LA SEQ ID NO: 4:
   (i) CARACTERISTIQUES DE LA SEQUENCE:
      (A) LONGUEUR: 541 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 3..536
      (D) AUTRES RENSEIGNEMENTS: /product= "Fragment C-ter de la chimere SAH-G.CSF"
(2) INFORMATION POUR LA SEQ ID NO: 5:
   (i) CARACTERISTIQUES DE LA SEQUENCE:
      (A) LONGUEUR: 2455 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 26..2389
      (D) AUTRES RENSEIGNEMENTS: /product= "Chimere G.CSF-Gly4-SAH en aval region prepro de SAH"
   (ix) CARACTERISTIQUE ADDITIONELLE:
      (A) NOM/CLE: misc_recomb
      (B) EMPLACEMENT: 620..631
      (D) AUTRES RENSEIGNEMENTS: /standard_name= "Linker PolyGly"
   (ix) CARACTERISTIQUE ADDITIONELLE:
      (A) NOM/CLE: misc feature
      (B) EMPLACEMENT: 106..111
      (D) AUTRES RENSEIGNEMENTS: /standard_name= "Site Apa I"
(2) INFORMATION POUR LA SEQ ID NO: 6:
   (i) CARACTERISTIQUES DE LA SEQUENCE:
      (A) LONGUEUR: 756 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 3..752
      (D) AUTRES RENSEIGNEMENTS: /product= "Fragment C-ter de la chimere SAH-Fv'"
   (ix) CARACTERISTIQUE ADDITIONELLE:
      (A) NOM/CLE: misc_recomb
      (B) EMPLACEMENT: 384..428
      (D) AUTRES RENSEIGNEMENTS: /standard_name= "Linker synthetique"

## Revendications

1. Polypeptide recombinant comportant une partie active dérivée d'un polypeptide ayant une activité thérapeutique, génétiquement couplée à une albumine ou à un variant de l'albumine, ledit variant de l'albumin conservant une haute demie-vie plasmatique, **caractérisé en ce que** le polypeptide ayant une activité thérapeutique est choisi parmi des hormones, des interférons, des interleukines, érythropoiétine, G-CSF et l'insuline.

2. Polypeptide selon la revendication 1 **caractérisé en ce que** le polypeptide ayant une activité thérapeutique est un polypeptide d'origine humaine.

3. Polypeptide selon l'une des revendications 1 ou 2 **caractérisé en ce que** la partie active présente une structure choisie parmi:
(a) la structure peptidique entière ou,
(b) un fragment de (a) ou une structure dérivée de (a) par modification structurale (mutation, substitution addition et/ou délétion d'un ou plusieurs résidus) et conservant une activité thérapeutique.

4. Polypeptide selon l'une des revendications 1 à 3 **caractérisé en ce que** l'albumine ou le variant de l'albumine possède une structure choisi parmi:
(a) l'albumine mature;
(b) l'albumine; et
(c) une structure dérivée de (a) ou (b) par modification structurale (mutation, substitution, addition et/ou délétion d'un ou plusieurs résidus) et conservant une haute demie-vie plasmatique.

5. Polypeptide selon l'une des revendications 1 à 4 **caractérisé en ce que** le polypeptide comprend une méthionine N-terminale.

6. Polypeptide selon l'une des revendications 1 à 5 **caractérisé en ce que** le polypeptide comprend un peptide de jonction.

7. Polypeptide selon l'une des revendications 1 à 6 **caractérisé en ce que** le polypeptide comprend un signal de sécrétion.

8. Polypeptide selon la revendication 7 **caractérisé en ce que** le signal de sécrétion est la séquence signal naturelle du polypeptide biologiquement actif.

9. Polypeptide selon l'une des revendications 1 à 8 **caractérisé en ce que** la partie active est couplée à l'extrémité N-terminale de l'albumine.

10. Polypeptide selon l'une des revendications 1 à 8 **caractérisé en ce que** la partie active est couplée à l'extrémité C-terminale de l'albumine.

11. Polypeptide selon l'une des revendications 1 à 10 **caractérisé en ce que** la partie active y est représenté plusieurs fois.

12. Séquence nucléotidique codant pour un polypeptide selon l'une quelconque des revendications 1 à 11.

13. Séquence nucléotidique selon la revendication 12 **caractérisée en ce qu'**elle comprend une séquence codant pour une séquence "leader" permettant la sécrétion du polypeptide exprimé.

14. Cassette d'expression comprenant une séquence nucléotidique selon l'une des revendications 12 ou 13 sous le contrôle d'une région d'initiation de la transcription et éventuellement d'une région de terminaison de la transcription.

15. Plasmide autoréplicatif comportant une cassette d'expression selon la revendication 14.

16. Cellule recombinante eucaryote ou procaryote dans laquelle a été inséré une séquence nucléotidique selon l'une des revendications 12 ou 13 ou une cassette d'expression selon la revendication 14 ou un plasmide selon la revendication 15.

17. Cellule recombinante selon la revendication 16 **caractérisée en ce qu'**il s'agit d'une levure, d'une cellule animale, d'un champignon ou d'une bactérie.

18. Cellule recombinante selon la revendication 17 **caractérisée en ce qu'**il s'agit d'une levure.

19. Cellule recombinante selon la revendication 18 **caractérisée en ce qu'**il s'agit d'une levure du genre Saccharomyces ou Kluyveromyces.

20. Procédé de préparation d'un polypeptide tel que défini dans l'une des revendications 1 à 11 **caractérisé en ce que** l'on cultive une cellule recombinante selon l'une des revendications 16 à 19 dans des conditions d'expression, et on récupère le polypeptide produit.

21. Composition pharmaceutique comprenant un ou plusieurs polypeptides selon l'une quelconque des revendications 1 à 11 ou préparés par un procédé selon la revendication 20.

22. Composition pharmaceutique comprenant une séquence nueléotidique selon l'une quelconque des revendications 12 à 14 utilisable en thérapie génique.

## Patentansprüche

1. Rekombinantes Polypeptid mit einem von einem Polypetid mit therapeutischer Aktivität erhaltenen aktiven Teil, der an ein Albumin oder eine Albuminvariante genetisch gekoppelt ist, wobei die Albuminvariante eine große Plasma-Halbwertszeit konserviert, **dadurch gekennzeichnet, dass** das Polypeptid mit therapeutischer Aktivität ausgewählt ist aus Hormonen, Interferonen, Interleukinen, Erythropoitin, G-CSF und Insulin.

2. Polypeptid nach Anspruch 1, **dadurch gekennzeichnet, dass** das Polypeptid mit therapeutischer Aktivität ein Polypeptid menschlichen Ursprungs ist.

3. Polypeptid nach Anspruch 1 oder 2, **dadurch gekennzeichnet, dass** der aktive Teil eine Struktur aufweist, die ausgewählt ist aus
(a) einer vollständigen Peptidstruktur oder
(b) einem Fragment von (a) oder einer durch strukturelle Modifikation (Mutation, Substitution und/oder Löschung von einem oder mehreren Rest(en)) und Konservierung der therapeutischen Aktivität von (a) erhaltenen Struktur.

4. Polypeptid nach einem der Ansprüche 1 bis 3, **dadurch gekennzeichnet, dass** das Albumin oder die Albuminvariante eine Struktur aufweist, die ausgewählt ist von
(a) reifem Albumin,
(b) Albumin; und
(c) einer von (a) oder (b) durch strukturelle Modifikation (Mutation, Substitution, Addition und/oder Löschung von einem oder mehreren Reten) und Konservierung der hohen Plasma-Halbwertszeit erhaltenen Struktur.

5. Polypeptid nach einem der Ansprüche 1 bis 4, **dadurch gekennzeichnet, dass** das Polypeptid ein N-terminales Methionin aufweist.

6. Polypeptid nach einem der Ansprüche 1 bis 5, **dadurch gekennzeichnet, dass** das Polypeptid ein Linkerpeptid aufweist.

7. Polypeptid nach einem der Ansprüche 1 bis 6, **dadurch gekennzeichnet, dass** das Polypeptid ein Sekretionssignal aufweist.

8. Polypeptid nach Anspruch 7, **dadurch gekennzeichnet, dass** das Sekretionssignal das natürliche Sekretionssignal des biologisch aktiven Polypeptids ist.

9. Polypeptid nach einem der Ansprüche 1 bis 8, **dadurch gekennzeichnet, dass** der aktive Teil an den N-Terminus des Albumins gekoppelt ist.

10. Polypeptid nach einem der Ansprüche 1 bis 8, **dadurch gekennzeichnet, dass** der aktive Teil an den C-Terminus des Albumins gekoppelt ist.

11. Polypeptid nach einem der Ansprüche 1 bis 10, **dadurch gekennzeichnet, dass** der aktive Teil mehrere Male vorhanden ist.

12. Nukleotidsequenz, welche ein Polypeptid nach einem der Ansprüche 1 bis 11 kodiert.

13. Nukleotidsequenz nach Anspruch 12, **dadurch gekennzeichnet, dass** sie eine Sequenz aufweist, die eine "Führer"-Sequenz kodiert, welche die Sekretion des expressivierten Polypeptids ermöglicht.

14. Expressionskassette, welche eine Nukleotidsequenz nach einem der Ansprüche 12 und 13 aufweist unter der Kontrolle eines Transkriptionsinitiierungsbereichs und optional eines Transkriptionsbeendigungsbereiches.

15. Selbstreplizierendes Plasmid, welches eine Expressionskassette nach Anspruch 14 aufweist.

16. Rekombinante eukaryotische oder prokaryotische Zelle, in welche eine Nukleotidsequenz nach Anspruch 12 oder 13 oder eine Expressionskassette nach Anspruch 14 oder ein Plasmid nach Anspruch 15 eingesetzt ist.

17. Rekombinante Zelle nach Anspruch 16, **dadurch gekennzeichnet, dass** es sich um eine Hefezelle, eine Tierzelle, eine Pilzzelle oder eine bakterielle Zelle handelt.

18. Rekombinante Zelle nach Anspruch 17, **dadurch gekennzeichnet, dass** es sich um eine Hefezelle handelt.

19. Rekombinante Zelle nach Anspruch 18, **dadurch gekennzeichnet, dass** es sich um eine Zelle der Art *Saccharomyces* oder *Kluyveromyces* handelt.

20. Verfahren zur Herstellung eines Polypeptides gemäß einem der Ansprüche 1 bis 11, **dadurch gekennzeichnet, dass** eine rekombinante Zelle nach einem der Ansprüche 16 bis 19 unter Expressionsbedingungen kultiviert wird und dass das Polypeptidprodukt geborgen wird.

21. Pharmazeutische Zusammensetzung mit einem oder mehreren Polypeptiden nach einem der Ansprüche 1 bis 11 oder hergestellt durch ein Verfahren nach Anspruch 20.

22. Pharmazeutische Zusammensetzung mit einer Nukleotidsequenz nach einem der Ansprüche 12 bis 14, welche in der Gentherapie einsetzbar ist.

## Claims

1. A recombinant polypeptide comprising an active part derived from a polypeptide having a therapeutic activity, genetically coupled to an albumin or a variant of albumin, wherein the variant of albumin conserves a high plasma half-life, **characterised in that** the polypeptide having a therapeutic activity is chosen from among hormones, interferons, interleukins, erythropoietin, G-CSF and insulin.

2. A polypeptide according to Claim 2 **characterised in that** the polypeptide having a therapeutic activity is a polypeptide of human origin.

3. A polypeptide according to Claim I or 2 **characterised in that** the active part has a structure chosen from
(a) an entire peptide structure or
(b) a fragment of (a) or a structure derived from (a) by structural modification (mutation, substitution, and/or deletion of one or more residues) and conservation of therapeutic activity.

4. A polypeptide according to one of Claims 1 to 3 **characterised in that** the albumin or albumin variant has a structure chosen from:
(a) mature albumin;
(b) albumin; and
(c) a structure derived from (a) or (b) by structural modification (mutation, substitution, addition and/or deletion of one or more residues) and conserving a high plasma half-life.

5. A polypeptide according to one of Claims 1 to 4 **characterised in that** the polypeptide comprises an N-terminal methionine.

6. A polypeptide according to one of Claims 1 to 5 **characterised in that** the polypeptide comprises a linker peptide.

7. A polypeptide according to one of Claims 1 to 6 **characterised in that** the polypeptide comprises a secretion signal.

8. A polypeptide according to Claim 8 **characterised in that** the secretion signal is the natural secretion signal of the biologically active polypeptide.

9. A polypeptide according to one of Claims 1 to 8 **characterised in that** the active part is coupled to the N-terminus of the albumin.

10. A polypeptide according to one of Claims 1 to 8 **characterised in that** the active part is coupled to the C-terminus of the albumin.

11. A polypeptide according to one of Claims 1 to 10 **characterised in that** the active part is represented several times.

12. Nucleotide sequence encoding a polypeptide according to one of Claims 1 to 11.

13. A nucleotide sequence according to Claim 12 **characterised in that** it comprises a sequence encoding a "leader" sequence permitting the secretion of the expressed polypeptide.

14. An expression cassette comprising a nucleotide sequence according to one of Claims 12 and 13 under the control of a transcription initiation region and optionally a transcription termination region.

15. A self-replicating plasmid comprising an expression cassette according to Claim 14.

16. A recombinant eukaryotic or prokaryotic cell in which has been inserted a nucleotide sequence according to Claim 12 or 13 or an expression cassette according to Claim 14 or a plasmid according to Claim 15.

17. A recombinant cell according to Claim 16 **characterised in that** it is a yeast, animal, fungal or bacterial cell.

18. A recombinant cell according to Claim 17 **characterised in that** it is a yeast cell.

19. A recombinant cell according to Claim 18 **characterised in that** it is a cell from the genus *Saccharomyces* or *Kluyveromyces.*

20. A process for preparing a polypeptide as defined in one of Claims 1 to 11 **characterised in that** one cultivates a recombinant cell according to one of Claims 16 to 19 under conditions for expression and one recovers the polypeptide product.

21. A pharmaceutical composition comprising one or more polypeptides according to Claims 1 to 11 or prepared by a process according to Claim 20.

22. A pharmaceutical composition comprising a nucleotide sequence according to one of Claims 12 to 14 usable in gene therapy.


### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: NOUVEAUX POLYPEPTIDES BIOLOGIQUEMENT ACTIFS, LEUR PREPARATION ET COMPOSITION PHARMACEUTIQUE LES CONTENANT.
   (iii) NOMBRE DE SEQUENCES: 6
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 1859 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 26..1855
      (D) AUTRES RENSEIGNEMENTS: /product= "Chimere de type SAH-Peptide"
   (ix) CARACTERISTIQUE ADDITIONELLE:
      (A) NOM/CLE: misc_feature
      (B) EMPLACEMENT: 1842..1848
      (D) AUTRES RENSEIGNEMENTS: /standard_name= "Site Mst II"
   (xi) DESCRIPTION DE LA SEQUENCE: SEQ ID NO: 1:
(2) INFORMATION POUR LA SEQ ID NO: 2:
   (i) CARACTERISTIQUES DE LA SEQUENCE:
      (A) LONGUEUR: 750 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 3..746
      (D) AUTRES RENSEIGNEMENTS: /product= "Fragment C-ter de la chimere SAH-vWF470"
   (xi) DESCRIPTION DE LA SEQUENCE: SEQ ID NO: 2:
   CC TTA GGC TTA ACC TGT GAA GCC TGC CAG GAG CCG GGA GGC CTG GTG      47
      Leu Gly Leu Thr Cys Glu Ala Cys Gln Glu Pro Gly Gly Leu Val
       1               5                  10                  15
   GTG CCT CCC ACA GAT GCC CCG GTG AGC CCC ACC ACT CTG TAT GTG GAG     95
   Val Pro Pro Thr Asp Ala Pro Val Ser Pro Thr Thr Leu Tyr Val Glu
                   20                  25                  30
   GAC ATC TCG GAA CCG CCG TTG CAC GAT TTC TAC TGC AGC AGG CTA CTG    143
   Asp Ile Ser Glu Pro Pro Leu His Asp Phe Tyr Cys Ser Arg Leu Leu
               35                  40                  45
   GAC CTG GTC TTC CTG CTG GAT GGC TCC TCC AGG CTG TCC GAG GCT GAG    191
   Asp Leu Val Phe Leu Leu Asp Gly Ser Ser Arg Leu Ser Glu Ala Glu
           50                  55                  60
   TTT GAA GTG CTG AAG GCC TTT GTG GTG GAC ATG ATG GAG CGG CTG CGC    239
   Phe Glu Val Leu Lys Ala Phe Val Val Asp Met Met Glu Arg Leu Arg
       65                  70                  75
   ATC TCC CAG AAG TGG GTC CGC GTG GCC GTG GTG GAG TAC CAC GAC GGC      287
   Ile Ser Gln Lys Trp Val Arg Val Ala Val Val Glu Tyr His Asp Gly
    80                  85                  90                  95
   TCC CAC GCC TAC ATC GGG CTC AAG GAC CGG AAG CGA CCG TCA GAG CTG      335
   Ser His Ala Tyr Ile Gly Leu Lys Asp Arg Lys Arg Pro Ser Glu Leu
                   100                 105                 110
   CGG CGC ATT GCC AGC CAG GTG AAG TAT GCG GGC AGC CAG GTG GCC TCC      383
   Arg Arg Ile Ala Ser Gln Val Lys Tyr Ala Gly Ser Gln Val Ala Ser
               115                 120                 125
   ACC AGC GAG GTC TTG AAA TAC ACA CTG TTC CAA ATC TTC AGC AAG ATC      431
   Thr Ser Glu Val Leu Lys Tyr Thr Leu Phe Gln Ile Phe Ser Lys Ile
           130                 135                 140
   GAC CGC CCT GAA GCC TCC CGC ATC GCC CTG CTC CTG ATG GCC AGC CAG      479
   Asp Arg Pro Glu Ala Ser Arg Ile Ala Leu Leu Leu Met Ala Ser Gln
       145                 150                 155
   GAG CCC CAA CGG ATG TCC CGG AAC TTT GTC CGC TAC GTC CAG GGC CTG      527
   Glu Pro Gln Arg Met Ser Arg Asn Phe Val Arg Tyr Val Gln Gly Leu
   160                 165                 170                 175
   AAG AAG AAG AAG GTC ATT GTG ATC CCG GTG GGC ATT GGG CCC CAT GCC      575
   Lys Lys Lys Lys Val Ile Val Ile Pro Val Gly Ile Gly Pro His Ala
                   180                 185                 190
   AAC CTC AAG CAG ATC CGC CTC ATC GAG AAG CAG GCC CCT GAG AAC AAG      623
   Asn Leu Lys Gln Ile Arg Leu Ile Glu Lys Gln Ala Pro Glu Asn Lys
               195                 200                 205
   GCC TTC GTG CTG AGC AGT GTG GAT GAG CTG GAG CAG CAA AGG GAC GAG      671
   Ala Phe Val Leu Ser Ser Val Asp Glu Leu Glu Gln Gln Arg Asp Glu
           210                 215                 220
   ATC GTT AGC TAC CTC TGT GAC CTT GCC CCT GAA GCC CCT CCT CCT ACT      719
   Ile Val Ser Tyr Leu Cys Asp Leu Ala Pro Glu Ala Pro Pro Pro Thr
       225                 230                 235
   CTG CCC CCC GAC ATG GCA CAA GTC TAAGCTT                              750
   Leu Pro Pro Asp Met Ala Gln Val
   240                 245
(2) INFORMATION POUR LA SEQ ID NO: 3:
   (i) CARACTERISTIQUES DE LA SEQUENCE:
      (A) LONGUEUR: 423 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: OUI
   (iii) ANTI-SENS: OUI
   (ix) CARACTERISTIQUE ADDITIONELLE:
      (A) NOM/CLE: CDS
      (B) EMPLACEMENT: 3..419
      (D) AUTRES RENSEIGNEMENTS: /product= "Fragment C-ter de la chimere SAH-UK1-135"
   (xi) DESCRIPTION DE LA SEQUENCE: SEQ ID NO: 3:
   CC TTA GGC TTA AGC AAT GAA CTT CAT CAA GTT CCA TCG AAC TGT GAC         47
      Leu Gly Leu Ser Asn Glu Leu His Gln Val Pro Ser Asn Cys Asp
        1               5                  10                  15
   TGT CTA AAT GGA GGA ACA TGT GTG TCC AAC AAG TAC TTC TCC AAC ATT        95
   Cys Leu Asn Gly Gly Thr Cys Val Ser Asn Lys Tyr Phe Ser Asn Ile
                    20                  25                  30
   CAC TGG TGC AAC TGC CCA AAG AAA TTC GGA GGG CAG CAC TGT GAA ATA       143
   His Trp Cys Asn Cys Pro Lys Lys Phe Gly Gly Gln His Cys Glu Ile
                35                  40                  45
   GAT AAG TCA AAA ACC TGC TAT GAG GGG AAT GGT CAC TTT TAC CGA GGA       191
   Asp Lys Ser Lys Thr Cys Tyr Glu Gly Asn Gly His Phe Tyr Arg Gly
            50                  55                  60
   AAG GCC AGC ACT GAC ACC ATG GGC CGG CCC TGC CTG CCC TGG AAC TCT       239
   Lys Ala Ser Thr Asp Thr Met Gly Arg Pro Cys Leu Pro Trp Asn Ser
        65                  70                  75
   GCC ACT GTC CTT CAG CAA ACG TAC CAT GCC CAC AGA TCT GAT GCT CTT       287
   Ala Thr Val Leu Gln Gln Thr Tyr His Ala His Arg Ser Asp Ala Leu
    80                  85                  90                  95
   CAG CTG GGC CTG GGG AAA CAT AAT TAC TGC AGG AAC CCA GAC AAC CGG       335
   Gln Leu Gly Leu Gly Lys His Asn Tyr Cys Arg Asn Pro Asp Asn Arg
                   100                 105                 110
   AGG CGA CCC TGG TGC TAT GTG CAG GTG GGC CTA AAG CCG CTT GTC CAA       383
   Arg Arg Pro Trp Cys Tyr Val Gln Val Gly Leu Lys Pro Leu Val Gln
               115                 120                 125
   GAG TGC ATG GTG CAT GAC TGC GCA GAT GGA AAA TAAGCTT                 423
   Glu Cys Met Val His Asp Cys Ala Asp Gly Lys
           130                 135
(2) INFORMATION POUR LA SEQ ID NO: 4:
   (i) CARACTERISTIQUES DE LA SEQUENCE:
      (A) LONGUEUR: 541 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 3..536
      (D) AUTRES RENSEIGNEMENTS: /product= "Fragment C-ter de la chimere SAH-G.CSF"
   (xi) DESCRIPTION DE LA SEQUENCE: SEQ ID NO: 4:
(2) INFORMATION POUR LA SEQ ID NO: 5:
   (i) CARACTERISTIQUES DE LA SEQUENCE:
      (A) LONGUEUR: 2455 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 26..2389
      (D) AUTRES RENSEIGNEMENTS: /product= "Chimere G.CSF-Gly4-SAH en aval region prepro de SAH"
   (ix) CARACTERISTIQUE ADDITIONELLE:
      (A) NOM/CLE: misc_recomb
      (B) EMPLACEMENT: 620..631
      (D) AUTRES RENSEIGNEMENTS: /standard_name= "Linker PolyGly"
   (ix) CARACTERISTIQUE ADDITIONELLE:
      (A) NOM/CLE: misc feature
      (B) EMPLACEMENT: 106..111
      (D) AUTRES RENSEIGNEMENTS: /standard_name= "Site Apa I"
   (xi) DESCRIPTION DE LA SEQUENCE: SEQ ID NO: 5:
   AAGCTTTACA ACAAATATAA AAACA ATG AAG TGG GTA ACC TTT ATT TCC CTT        52
                               Met Lys Trp Val Thr Phe Ile Ser Leu
                                1               5
   CTT TTT CTC TTT AGC TCG GCT TAT TCC AGG GGT GTG TTT CGT CGA ACC       100
   Leu Phe Leu Phe Ser Ser Ala Tyr Ser Arg Gly Val Phe Arg Arg Thr
    10                  15                  20                  25
   CCC CTG GGC CCT GCC AGC TCC CTG CCC CAG AGC TTC CTG CTC AAG TGC       148
   Pro Leu Gly Pro Ala Ser Ser Leu Pro Gln Ser Phe Leu Leu Lys Cys
                    30                  35                  40
   TTA GAG CAA GTG AGG AAG ATC CAG GGC GAT GGC GCA GCG CTC CAG GAG       196
   Leu Glu Gln Val Arg Lys Ile Gln Gly Asp Gly Ala Ala Leu Gln Glu
                45                  50                  55
   AAG CTG TGT GCC ACC TAC AAG CTG TGC CAC CCC GAG GAG CTG GTG CTG       244
   Lys Leu Cys Ala Thr Tyr Lys Leu Cys His Pro Glu Glu Leu Val Leu
            60                  65                  70
   CTC GGA CAC TCT CTG GGC ATC CCC TGG GCT CCC CTG AGC TCC TGC CCC       292
   Leu Gly His Ser Leu Gly Ile Pro Trp Ala Pro Leu Ser Ser Cys Pro
        75                  80                  85
   AGC CAG GCC CTG CAG CTG GCA GGC TGC TTG AGC CAA CTC CAT AGC GGC       340
   Ser Gln Ala Leu Gln Leu Ala Gly Cys Leu Ser Gln Leu His Ser Gly
    90                  95                 100                 105
   CTT TTC CTC TAC CAG GGG CTC CTG CAG GCC CTG GAA GGG ATA TCC CCC       388
   Leu Phe Leu Tyr Gln Gly Leu Leu Gln Ala Leu Glu Gly Ile Ser Pro
                   110                 115                 120
   GAG TTG GGT CCC ACC TTG GAC ACA CTG CAG CTG GAC GTC GCC GAC TTT       436
   Glu Leu Gly Pro Thr Leu Asp Thr Leu Gln Leu Asp Val Ala Asp Phe
               125                 130                 135
   GCC ACC ACC ATC TGG CAG CAG ATG GAA GAA CTG GGA ATG GCC CCT GCC       484
   Ala Thr Thr Ile Trp Gln Gln Met Glu Glu Leu Gly Met Ala Pro Ala
           140                 145                 150
   CTG CAG CCC ACC CAG GGT GCC ATG CCG GCC TTC GCC TCT GCT TTC CAG      532
   Leu Gln Pro Thr Gln Gly Ala Met Pro Ala Phe Ala Ser Ala Phe Gln
       155                 160                 165
   CGC CGG GCA GGA GGG GTC CTG GTT GCT AGC CAT CTG CAG AGC TTC CTG      580
   Arg Arg Ala Gly Gly Val Leu Val Ala Ser His Leu Gln Ser Phe Leu
   170                 175                 180                 185
   GAG GTG TCG TAC CGC GTT CTA CGC CAC CTT GCG CAG CCC GGT GGA GGC      628
   Glu Val Ser Tyr Arg Val Leu Arg His Leu Ala Gln Pro Gly Gly Gly
                   190                 195                 200
   GGT GAT GCA CAC AAG AGT GAG GTT GCT CAT CGG TTT AAA GAT TTG GGA      676
   Gly Asp Ala His Lys Ser Glu Val Ala His Arg Phe Lys Asp Leu Gly
               205                 210                 215
   GAA GAA AAT TTC AAA GCC TTG GTG TTG ATT GCC TTT GCT CAG TAT CTT      724
   Glu Glu Asn Phe Lys Ala Leu Val Leu Ile Ala Phe Ala Gln Tyr Leu
           220                 225                 230
   CAG CAG TGT CCA TTT GAA GAT CAT GTA AAA TTA GTG AAT GAA GTA ACT      772
   Gln Gln Cys Pro Phe Glu Asp His Val Lys Leu Val Asn Glu Val Thr
       235                 240                 245
   GAA TTT GCA AAA ACA TGT GTT GCT GAT GAG TCA GCT GAA AAT TGT GAC      820
   Glu Phe Ala Lys Thr Cys Val Ala Asp Glu Ser Ala Glu Asn Cys Asp
   250                 255                 260                 265
   AAA TCA CTT CAT ACC CTT TTT GGA GAC AAA TTA TGC ACA GTT GCA ACT      868
   Lys Ser Leu His Thr Leu Phe Gly Asp Lys Leu Cys Thr Val Ala Thr
                   270                 275                 280
   CTT CGT GAA ACC TAT GGT GAA ATG GCT GAC TGC TGT GCA AAA CAA GAA      916
   Leu Arg Glu Thr Tyr Gly Glu Met Ala Asp Cys Cys Ala Lys Gln Glu
               285                 290                 295
   CCT GAG AGA AAT GAA TGC TTC TTG CAA CAC AAA GAT GAC AAC CCA AAC      964
   Pro Glu Arg Asn Glu Cys Phe Leu Gln His Lys Asp Asp Asn Pro Asn
           300                 305                 310
   CTC CCC CGA TTG GTG AGA CCA GAG GTT GAT GTG ATG TGC ACT GCT TTT     1012
   Leu Pro Arg Leu Val Arg Pro Glu Val Asp Val Met Cys Thr Ala Phe
       315                 320                 325
   CAT GAC AAT GAA GAG ACA TTT TTG AAA AAA TAC TTA TAT GAA ATT GCC     1060
   His Asp Asn Glu Glu Thr Phe Leu Lys Lys Tyr Leu Tyr Glu Ile Ala
   330                 335                 340                 345
   AGA AGA CAT CCT TAC TTT TAT GCC CCG GAA CTC CTT TTC TTT GCT AAA     1108
   Arg Arg His Pro Tyr Phe Tyr Ala Pro Glu Leu Leu Phe Phe Ala Lys
                   350                 355                 360
   AGG TAT AAA GCT GCT TTT ACA GAA TGT TGC CAA GCT GCT GAT AAA GCT     1156
   Arg Tyr Lys Ala Ala Phe Thr Glu Cys Cys Gln Ala Ala Asp Lys Ala
               365                 370                 375
   GCC TGC CTG TTG CCA AAG CTC GAT GAA CTT CGG GAT GAA GGG AAG GCT     1204
   Ala Cys Leu Leu Pro Lys Leu Asp Glu Leu Arg Asp Glu Gly Lys Ala
           380                 385                 390
   TCG TCT GCC AAA CAG AGA CTC AAG TGT GCC AGT CTC CAA AAA TTT GGA     1252
   Ser Ser Ala Lys Gln Arg Leu Lys Cys Ala Ser Leu Gln Lys Phe Gly
       395                 400                 405
   GAA AGA GCT TTC AAA GCA TGG GCA GTA GCT CGC CTG AGC CAG AGA TTT     1300
   Glu Arg Ala Phe Lys Ala Trp Ala Val Ala Arg Leu Ser Gln Arg Phe
   410                 415                 420                 425
   CCC AAA GCT GAG TTT GCA GAA GTT TCC AAG TTA GTG ACA GAT CTT ACC     1348
   Pro Lys Ala Glu Phe Ala Glu Val Ser Lys Leu Val Thr Asp Leu Thr
                   430                 435                 440
   AAA GTC CAC ACG GAA TGC TGC CAT GGA GAT CTG CTT GAA TGT GCT GAT     1396
   Lys Val His Thr Glu Cys Cys His Gly Asp Leu Leu Glu Cys Ala Asp
               445                 450                 455
   GAC AGG GCG GAC CTT GCC AAG TAT ATC TGT GAA AAT CAA GAT TCG ATC     1444
   Asp Arg Ala Asp Leu Ala Lys Tyr Ile Cys Glu Asn Gln Asp Ser Ile
           460                 465                 470
   TCC AGT AAA CTG AAG GAA TGC TGT GAA AAA CCT CTG TTG GAA AAA TCC     1492
   Ser Ser Lys Leu Lys Glu Cys Cys Glu Lys Pro Leu Leu Glu Lys Ser
       475                 480                 485
   CAC TGC ATT GCC GAA GTG GAA AAT GAT GAG ATG CCT GCT GAC TTG CCT     1540
   His Cys Ile Ala Glu Val Glu Asn Asp Glu Met Pro Ala Asp Leu Pro
   490                 495                 500                 505
   TCA TTA GCT GCT GAT TTT GTT GAA AGT AAG GAT GTT TGC AAA AAC TAT     1588
   Ser Leu Ala Ala Asp Phe Val Glu Ser Lys Asp Val Cys Lys Asn Tyr
                   510                 515                 520
   GCT GAG GCA AAG GAT GTC TTC CTG GGC ATG TTT TTG TAT GAA TAT GCA    1636
   Ala Glu Ala Lys Asp Val Phe Leu Gly Met Phe Leu Tyr Glu Tyr Ala
               525                 530                 535
   AGA AGG CAT CCT GAT TAC TCT GTC GTA CTG CTG CTG AGA CTT GCC AAG    1684
   Arg Arg His Pro Asp Tyr Ser Val Val Leu Leu Leu Arg Leu Ala Lys
           540                 545                 550
   ACA TAT GAA ACC ACT CTA GAG AAG TGC TGT GCC GCT GCA GAT CCT CAT    1732
   Thr Tyr Glu Thr Thr Leu Glu Lys Cys Cys Ala Ala Ala Asp Pro His
       555                 560                 565
   GAA TGC TAT GCC AAA GTG TTC GAT GAA TTT AAA CCT CTT GTG GAA GAG    1780
   Glu Cys Tyr Ala Lys Val Phe Asp Glu Phe Lys Pro Leu Val Glu Glu
   570                 575                 580                 585
   CCT CAG AAT TTA ATC AAA CAA AAT TGT GAG CTT TTT GAG CAG CTT GGA    1828
   Pro Gln Asn Leu Ile Lys Gln Asn Cys Glu Leu Phe Glu Gln Leu Gly
                   590                 595                 600
   GAG TAC AAA TTC CAG AAT GCG CTA TTA GTT CGT TAC ACC AAG AAA GTA    1876
   Glu Tyr Lys Phe Gln Asn Ala Leu Leu Val Arg Tyr Thr Lys Lys Val
               605                 610                 615
   CCC CAA GTG TCA ACT CCA ACT CTT GTA GAG GTC TCA AGA AAC CTA GGA    1924
   Pro Gln Val Ser Thr Pro Thr Leu Val Glu Val Ser Arg Asn Leu Gly
           620                 625                 630
   AAA GTG GGC AGC AAA TGT TGT AAA CAT CCT GAA GCA AAA AGA ATG CCC    1972
   Lys Val Gly Ser Lys Cys Cys Lys His Pro Glu Ala Lys Arg Met Pro
       635                 640                 645
   TGT GCA GAA GAC TAT CTA TCC GTG GTC CTG AAC CAG TTA TGT GTG TTG    2020
   Cys Ala Glu Asp Tyr Leu Ser Val Val Leu Asn Gln Leu Cys Val Leu
   650                 655                 660                 665
   CAT GAG AAA ACG CCA GTA AGT GAC AGA GTC ACC AAA TGC TGC ACA GAA    2068
   His Glu Lys Thr Pro Val Ser Asp Arg Val Thr Lys Cys Cys Thr Glu
                   670                 675                 680
   TCC TTG GTG AAC AGG CGA CCA TGC TTT TCA GCT CTG GAA GTC GAT GAA    2116
   Ser Leu Val Asn Arg Arg Pro Cys Phe Ser Ala Leu Glu Val Asp Glu
               685                 690                 695
   ACA TAC GTT CCC AAA GAG TTT AAT GCT GAA ACA TTC ACC TTC CAT GCA    2164
   Thr Tyr Val Pro Lys Glu Phe Asn Ala Glu Thr Phe Thr Phe His Ala
           700                 705                 710
   GAT ATA TGC ACA CTT TCT GAG AAG GAG AGA CAA ATC AAG AAA CAA ACT    2212
   Asp Ile Cys Thr Leu Ser Glu Lys Glu Arg Gln Ile Lys Lys Gln Thr
       715                 720                 725
   GCA CTT GTT GAG CTT GTG AAA CAC AAG CCC AAG GCA ACA AAA GAG CAA    2260
   Ala Leu Val Glu Leu Val Lys His Lys Pro Lys Ala Thr Lys Glu Gln
   730                 735                 740                 745
   CTG AAA GCT GTT ATG GAT GAT TTC GCA GCT TTT GTA GAG AAG TGC TGC    2308
   Leu Lys Ala Val Met Asp Asp Phe Ala Ala Phe Val Glu Lys Cys Cys
                   750                 755                 760
   AAG GCT GAC GAT AAG GAG ACC TGC TTT GCC GAG GAG GGT AAA AAA CTT    2356
   Lys Ala Asp Asp Lys Glu Thr Cys Phe Ala Glu Glu Gly Lys Lys Leu
               765                 770                 775
   GTT GCT GCA AGT CAA GCT GCC TTA GGC TTA TAACATCACA TTTAAAAGCA      2406
   Val Ala Ala Ser Gln Ala Ala Leu Gly Leu
           780                 785
   TCTCAGCCTA CCATGAGAAT AAGAGAAAGA AAATGAAGAT CAAAAGCTT              2455
(2) INFORMATION POUR LA SEQ ID NO: 6:
   (i) CARACTERISTIQUES DE LA SEQUENCE:
      (A) LONGUEUR: 756 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 3..752
      (D) AUTRES RENSEIGNEMENTS: /product= "Fragment C-ter de la chimere SAH-Fv'"
   (ix) CARACTERISTIQUE ADDITIONELLE:
      (A) NOM/CLE: misc recomb
      (B) EMPLACEMENT: 384..428
      (D) AUTRES RENSEIGNEMENTS: /standard name= "Linker synthetique"
   (xi) DESCRIPTION DE LA SEQUENCE: SEQ ID NO: 6: